# EUROPEAN PATENT APPLICATION

(11) **EP 4 140 542 A1**
(43) Date of publication of application: **01.03.2023**
(21) Application number: 21306141.9
(22) Date of filing: 24.08.2021
(51) Int. Cl.: A61P 21/00, A61K 31/336, A61P 25/16, A61P 27/06, A61P 35/00, C07D 303/48, C12Q 1/37, C07D 403/04, C07D 405/10, C07D 417/12

(54) **NEW VASHS INHIBITORS, CONJUGATES THEREOF AND THEIR USES AS DRUGS OR AS RESEARCH TOOLS**

(71) Applicant: CENTRE NATIONAL DE LA RECHERCHE SCIENTIFIQUE (CNRS), 75016 Paris (FR); UNIVERSITE DE MONTPELLIER, 34090 Montpellier (FR); Ecole Nationale Supérieure de Chimie de Montpellier, 34090 Montpellier (FR); Mt Act, 34960 Montpellier Cedex 02 (FR)
(72) Inventor: ROGOWSKI, Krzysztof, 34070 MONTPELLIER (FR); AMBLARD-CAUSSIL, Muriel, 34730 SAINT VINCENT DE BARBEYRARGUES (FR); VAN DER LAAN, Siem, 34190 CAZILHAC (FR); VEZENKOV, Lubomir, 34090 MONTPELLIER (FR); MARCELLIN, Guillaume, 34790 GRABELS (FR); LANNAY, Yoann, 34380 MAS DE LONDRES (FR); HACHED, Khaled, 34090 MONTPELLIER (FR)
(74) Representative: Regimbeau

(57) **Abstract**

The present invention concerns a compound of formula (I) : 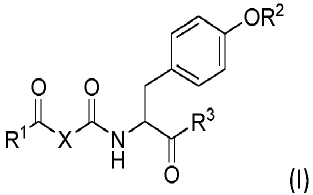 or a pharmaceutically acceptable salt and/or solvate thereof, wherein X, R1, R2, R3 are defined, a conjugate thereof and their uses as drug or research tools.

## Description

### Field of the invention

The present invention relates to new VASH inhibitors, particularly useful in the prevention and/or the treatment of VASH-peptidase associated disorders and/or as research tools. The present invention also relates to a conjugate of said VASH inhibitors with a biomolecule and its use as drug or as research tool.

### Background of the invention

Microtubules (MTs) are the major types of cytoskeletal elements, and are particularly abundant in neurons. They are formed by polymerization of alpha- and beta-tubulin heterodimers. Within a cell, MTs have the ability to grow and shrink, a phenomenon that has been termed dynamic instability. The dynamic behavior of MTs allows them to support numerous cellular processes including intracellular transport, cell motility, cell division and cell morphogenesis. The functional diversity of microtubules is due to the various and numerous post-translational modifications (PTM) that affect tubulins. Known PTMs include acetylation, polyglutamylation, polyglycylation, phosphorylation, and tyrosination/detyrosination cycles. These modifications have an impact on microtubule dynamics, their organization, and their interactions with other cellular compounds and compartments.

Dysfunctions of MTs are associated with neurodevelopmental disorders as well as neurodegeneration. Well-described dysfunctions have been linked to mutations either in tubulins, the building blocks of MTs, or MT-associated proteins (MAPs) that regulate MT functions. The binding of many MAPs is mediated by the C-terminal tails of α- and β-tubulin that protrude at the surface of MTs known to be heavily subjected to various posttranslational modifications. The most abundant tubulin modifications in neurons is polyglutamylation, which generates multiple glutamate sidechains of variable length on both α- and β-tubulin C-termini. The negatively charged glutamates are added to the C-terminal tails that provide the binding sites for various MT-associated proteins (MAPs) and molecular motors. Accordingly, polyglutamylation has been shown to regulate the activity or binding of a number of MT-interacting proteins. Early studies have suggested that the binding of neuronal MAPs such as tau, a key player in Alzheimer's disease, is regulated by MT polyglutamylation. More recent reports have demonstrated that the activity of MT severing enzymes such as spastin and katanin, which play important roles in axonal growth and plasticity, is also controlled by this modification. Furthermore, polyglutamylation appears to regulate the velocity and processivity of KIF1A kinesin motor protein, which is involved in the delivery of synaptic vesicles and as such regulates synaptic transmission. Taken together tubulin polyglutamylation controls various important neuronal processes. Polyglutamylation is reversible and catalyzed by the enzymes belonging to the Tubulin Tyrosine Ligase Like (TTLL) family. In contrast, the reverse enzymes are members of the Cytosolic Carboxypeptidase (CCP) family. As such, the overall level of tubulin polyglutamylation in a particular cell is established as a result of the competition between both activities.

The first tubulin modification to be discovered was detyrosination, which is specific to alpha-tubulin and consists of the removal of the very C-terminal tyrosine residue of the MT. The tyrosination/detyrosination cycle is particularly involved with microtubule regulation in the neuronal cells, muscle cells, and dividing cells. Altered microtubule detyrosination is thus involved in disorders such as neurodegenerative diseases, neuronal regeneration disorders, cancers, muscular dystrophies, heart diseases, vascular disorders, retinal degeneration, infertility or ciliopathies.

Although detyrosination has been discovered almost half a century ago, the enzymes involved in its generation, which possess tubulin carboxypeptidase (TCP) activity, have been identified only very recently (Nieuwenhuis J. et al. Science, 2017, 358(6369):1453-1456; Aillaud C. et al. Science, 2017, 358(6369:1448-1453). The members of the vasohibin family VASH1 and VASH2 are the enzymes that catalyze this modification. Recent research notably led to the identification of reversible and irreversible inhibitors for this protein enzymatic activity which are of particular interest for the treatment of neurodegenerative disorders including but not limited to tauopathies (e.g. Alzheimer's disease, frontotemporal dementia, corticobasal dementia and others) as well as the above recited disorders (see WO 2019/016259).

The identification of VASHs as tubulin detyrosinases has been made possible by employing a high affinity purification step using custom-designed inhibitors compatible with click chemistry (Aillaud, C. et al., Science. 2017, 358(6369):1448-1453). The most efficient among said inhibitors, referred as compound EPO-Y (Aillaud et al. 2017), is composed of an active group called transepoxysuccinate (TES) ethyl ester linked to a tyrosine residue, represented as follows :

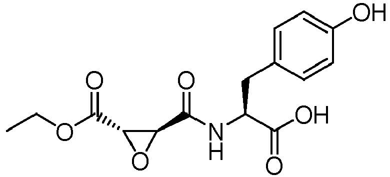

EPO-Y (also termed Epo-Y) is an irreversible VASHs inhibitor initially considered as potential candidate for the treatment of disorders involving altered microtubule detyrosination. However, this compound shows a moderate IC₅₀ of about of 15 µM *in cellulo.* In light of potential biomedical applications, high specificity and potency for VASHs are required for an ideal inhibitor. A small and highly electrophile epoxide molecule such as EPO-Y might interact with free thiols present on other proteins and in cysteine protease active sites especially at high µM range concentrations. Additionally, it could react with nucleophilic lysine residues in the tyrosine kinases' active sites, resulting in a loss of specificity. Strikingly, the only other reported inhibitor of detyrosination, called parthenolide, which was identified in the past via a cell-based screen (Fonrose et al. Cancer Res 2007; 67(7):3371-8), does not inhibit VASHs *in vitro* even at highly elevated concentrations (Hotta T. et al., Curr. Biol., 2021, 31(4):900-907). This suggests that the observed *in cellulo* effect of parthenolide is not mediated by a direct inhibition of VASHs.

Thus, there is a need for improving the existing VASHs inhibitor specificity, potency and bioavailability in order to provide a suitable candidate useful in the treatment of disorders involving altered microtubule detyrosination. In this invention, we describe highly efficient low nanomolar to picomolar, *in cellulo* VASHs inhibitors as potential drugs for the treatment of neurodegenerative diseases.

### Summary of the present invention

Using medicinal chemistry, libraries of compounds were generated, measured the effect in an ideally predictive *in vitro* system and used bioinformatics to model and understand the structural properties responsible for the variations in biological activities of the compounds. Hence, the inventors have developed new VASHs inhibitors that present highly enhanced in *vitro* and *in cellulo* activity as compared to EPO-Y.

The present invention thus relates to a compound of the following formula (I) : 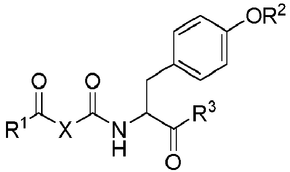 or a pharmaceutically acceptable salt and/or solvate thereof, in which
X is 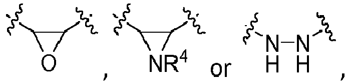
R¹ is O-C₁-C₆alkyl, O-C₂-C₆ alkenyl, NH-OH, or 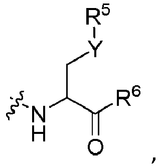
R² is H, a C₁-C₆ aliphatic chain, aryl, heteroaryl or C₁-C₆ alkyl-aryl, wherein up to 4 methylene units of said aliphatic chain are optionally replaced by O, C(O), NH or N-C₁-C₆alkyl, said aliphatic chain, aryl, heteroaryl or alkyl-aryl being optionally substituted,
R³ is OH, O-C₁-C₆ aliphatic chain, O-aryl, O-C₁-C₆ alkyl-aryl, O-heteroaryl, O-C₁-C₆ alkyl-heteroaryl or NHOH, wherein up to 4 methylene units of said aliphatic chain are optionally replaced by O, C(O), NH or, N-C₁-C₆alkyl, said aliphatic chain, aryl, heteroaryl, alkyl-heteroaryl or alkyl-aryl being optionally substituted,
R⁴ is H or a C₁-C₁₂ aliphatic chain wherein up to 4 methylene units are optionally replaced by O, C(O), NH or N-C₁-C₆alkyl, said C₁-C₁₂ aliphatic chain being optionally substituted,
Y is ―(CH₂)ₘ― or 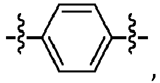
R⁵ is OH, O-C₁-C₆ alkyl, C₂-C₆ alkenyl, C₂-C₆ alkynyl, aryl, heteroaryl, O-C₁-C₆ alkyl-aryl, O-C₁-C₆ alkyl-heteroaryl, C(O)OH, C(O)O-C₁-C₆ alkyl or C(O)NHOH, said alkyl, alkenyl, alkynyl, aryl, heteroaryl, alkyl-aryl or alkyl-heteroaryl being optionally substituted,
R⁶ is OH, O-C₁-C₆ alkyl, NH-OH or NH-CH(R⁷)-(CH₂)ₙ-R⁸, said alkyl being optionally substituted,
R⁷ is H, C₁-C₆ alkyl, C₂-C₆ alkenyl, C₂-C₆ alkynyl, aryl, heteroaryl, C₁-C₆ alkyl-aryl or C₁-C₆ alkyl-heteroaryl,
R⁸ is C(O)NH₂, C(O)NH-C₁-C₆ alkyl, aryl, heteroaryl, SH, NH₂ or S-C₁-C₆alkyl, and m and n are each independently an integer ranging from 0 to 6, provided that when X is 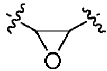 and R³ is OH, R¹ is not O-C₁-C₆ alkyl.

According to another aspect, the present invention relates to a pharmaceutical composition comprising at least one compound of formula (I) as defined above and at least one pharmaceutically acceptable excipient.

According to another aspect, the present invention also relates to the compound of formula (I) or the pharmaceutical composition as defined above for use as a drug.

The present invention also relates to the compound of formula (I) for use as a research tool.

According to another aspect, the present invention also relates to a conjugate comprising a compound of formula (I) linked to a biomolecule.

According to another aspect, the present invention relates to the conjugate as defined above for use as drug or as research tool.

### Detailed description

### Definitions

The term "stereoisomers" used in this invention refers to configurational stereoisomers and more particularly to optical isomers.

In the present invention, the optical isomers result in particular from the different position in space of the substituents attached to X. The carbon atoms or the nitrogen atoms of the X group to which the substituents are attached thus represent chiral or asymmetric centres. Optical isomers that are not mirror images of one another are thus designated as "diastereoisomers", and optical isomers, which are non-superimposable mirror images are designated as "enantiomers".

An equimolar mixture of two enantiomers of a chiral compound is designated as a racemic mixture or racemate.

In the context of the present invention, depending of the position of the substituents attached to the X group, the compound of the present invention may be a diastereoisomer of configuration (S,S), of configuration (R,R), of configuration (S,R) or of configuration (R,S) as illustrated hereafter:

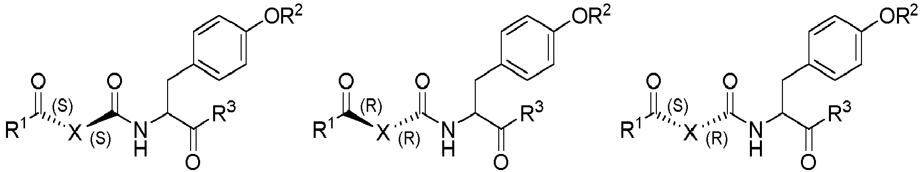

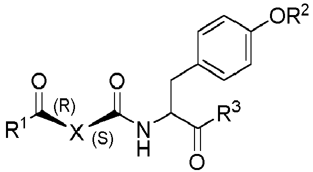

When the position of the substituents is not specified in the compound, said compound corresponds to any one of the above diastereoisomers or to a mixture of said diastereoisomers.

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt and/or solvate" is intended to mean, in the framework of the present invention, a salt and/or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L25 tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

The term "halogen", as used in the present invention, refers to a fluorine, bromine, chlorine or iodine atom.

The term "Cₓ-C_{y} aliphatic chain" designates a linear or branched hydrocarbon chain, completely saturated or containing one or more unsaturations, but not aromatic, comprising from x to y carbon atoms, notably from 1 to 12 carbon atoms, preferably from 1 to 6 carbon atoms. According to the present invention, the term "aliphatic chain" includes substituted or unsubstituted, linear or branched, alkyl, alkenyl or alkynyl groups.

The term "C₁-C₆ alkyl", as used in the present invention, refers to a straight or branched monovalent saturated hydrocarbon chain containing from 1 to 6 carbon atoms including, but not limited to, methyl, ethyl, n-propyl, iso-propyl, n-butyl, iso-butyl, sec-butyl, *t*-butyl, n-pentyl, n-hexyl, and the like.

The term "C₂-C₆ alkenyl", as used in the present invention, refers to a straight or branched monovalent unsaturated hydrocarbon chain containing from 2 to 6 carbon atoms and comprising at least one double bond including, but not limited to, ethenyl, propenyl, butenyl, pentenyl, hexenyl and the like.

The term "C₂-C₆ alkynyl", as used in the present invention, refers to a straight or branched monovalent unsaturated hydrocarbon chain containing from 2 to 6 carbon atoms and comprising at least one triple bond including, but not limited to, ethynyl, propynyl, propynyl, butynyl, pentynyl, hexynyl and the like.

The term "aryl", as used in the present invention, refers to an aromatic hydrocarbon group preferably comprising from 6 to 12 carbon atoms and comprising one or more fused rings, such as, for example but not limited to, a phenyl or naphthyl group. Advantageously, it is a phenyl group.

The term "heteroaryl", as used in the present invention, refers to an aromatic group comprising one or several, notably one or two, fused hydrocarbon cycles in which one or several, notably one to four, advantageously one or two, carbon atoms each have been replaced with a heteroatom selected from a sulfur atom, an oxygen atom and a nitrogen atom, preferably selected from an oxygen atom and a nitrogen atom. It can be a furyl, thienyl, pyrrolyl, pyridyl, oxazolyl, isoxazolyl, thiazolyle, isothiazolyl, imidazolyl, pyrazolyl, oxadiazolyl, thiadiazolyl, triazolyl, tetrazolyl, pyridazinyl, pyrimidinyl, pyrazinyl, triazinyl, quinolyl, isoquinolyl, quinoxalyl or indyl.

The term "C₁-C₆-alkyl aryl" or "C₁-C₆-alkyl heteroaryl" as used in the present invention, refers to an alkyl group as defined above substituted respectively by an aryl or a heteroaryl group as defined above. Advantageously, a "C₁-C₆-alkyl aryl" is a benzyl group.

In the context of the present invention, "optionally substituted" means that the group in question is optionally substituted with one or more substituents which may be selected in particular from halogen, C₁-C₆ alkyl, C₁-C₆ haloalkyl, C₂-C₆ alkene, C₂-C₆ alkyne, aryl, N₃, oxo, NR^{a}R^{b}, COR^{c}, CO₂R^{d}, CONR^{e}R^{f}, OR^{g}, N⁺R^{h}RⁱR^{j}, CN and NO₂ wherein R^{a} to R^{j} are, independently of one another, H, C₁-C₆ alkyl or aryl, preferably H or C₁-C₆ alkyl.

The term "C₁-C₆ haloalkyl" refers to a C₁-C₆ alkyl chain as defined above wherein one or more hydrogen atoms are replaced by a halogen atom selected from fluorine, chlorine, bromine or iodine, preferably a fluorine atom. For example, it is a CF₃ group.

In the context of the present invention, "unsaturated" means that the hydrocarbon chain may contain one or more unsaturation(s), i.e. a double bond C=C or a triple bond C=C, advantageously one.

The term "pharmaceutical composition" is meant in the framework of the present invention a composition having preventive and curative properties.

The term "peptide coupling" refers to a chemical reaction between an amine function and a carboxylic acid function. The peptide coupling will be advantageously carried out in the presence of a coupling agent, such as diisopropylcarbodiimide (DIC), dicyclohexylcarbodiimide (DCC), 1-(3-dimethylaminopropyl)-3-ethylcarbodiimide hydrochloride (EDC), carbonyldiimidazole (CDI), hexafluorophosphate 2-(1H-benzotriazole-1-yl)-1,1,3,3-tetramethyluronium (HBTU), tetrafluoroborate 2-(1H-benzotriazole-1-yl)-1,1,3,3-tetramethyluronium (TBTU), hexafluorophosphate O-(7-azobenzotriazol-1-yl)-1,1,3,3-tetramethyluronium (HATU), (benzotriazol-1-yloxy)tripyrrolodinophosphonium hexafluorophosphate (PyBOP), 7-Azabenzotriazol-1-yloxy)tripyrrolidinophosphonium hexafluorophosphate (PyAOP) or propylphosphonic anhydride; optionally associated with an additive or a base, such as N-hydroxy-succinimide (NHS), N-hydroxy-benzotriazole (HOBt), 3,4-dihydro-3-hydroxy-4-oxo-1,2,3-benzotriazole (HOOBt), 1-hydroxy-7-azabenzotriazole (HAt), N-hydroxysylfosuccinimide (sulfo NHS), dimethylaminopyridine (DMAP), diisopropylethylamine (DIEA) or N-methylmorpholine (NMM).

The term "VASH (enzyme)", as used in the present invention, refers to a tubulin carboxypeptidase enzyme (TCPase) involved in microtubules detyrosination mechanisms associated with neurodegenerative disorders, mental disorders, neurological disorders, ciliopathies, cancers and muscular dystrophies.

The term "biomolecule" refers to a molecule having biological properties. In the context of the present invention, it refers to a protein, a peptide, a biomarker, such as a photolabeling agent, or a E3 ubiquitin ligase recruiter including but not limited to Thalidomide, VH032, VH101, dBET1, dFKBP12, QCA570, PROTAC6, ZNL-02-096 or d9A-2.

The term "PROTAC", as used in the present invention, is shortened form of PROteolysis Targeting Chimeras. A "PROTAC E3 ligase recruiter" is a conjugate comprising two ligands connected by a linker useful as tool for selective and complete protein degradation. One of the ligands is intended to bind the protein targeted for degradation, while the other should target an ubiquitin ligase. As a consequence, PROTAC E3 ligase recruiter can bring into proximity the protein targeted for degradation and the ligase which leads to the (poly)ubiquitination, i.e. the addition of a single or multiple ubiquitins, which are small (8.6 kDa) regulatory proteins, of the target protein to a substrate protein. The (poly)ubiquitin tag, attached onto the protein targeted for degradation, sends it for degradation by the proteasome. Contrary to 'conventional' inhibitors, PROTAC E3 ligase recruiter induces selective proteolysis, thereby suppressing all its biological functions. In the context of the present invention, the protein targeted for degradation is the VASH enzyme. Thus, the ligand of the PROTAC E3 ligase recruiter intended to bind the VASH enzyme is a fragment of a compound of formula (I) and the ligand intended to target the ubiquitin ligase is typically the Thalidomide.

The term "prodrug" relates to a pharmacologically inactive derivative of an active drug and undergoes *in vivo* biotransformation to release the active drug by chemical or enzymatic cleavages. Prodrugs can offer many advantages over parent drugs such as increased solubility, enhanced stability, improved bioavailability, reduced side effects, and better selectivity. Activation of prodrugs can involve many enzymes including but not limited to oxidoreductases like CYP450 and DT-diaphorase as well as hydrolytic enzymes like carboxylesterase and β-glucuronidase.

### Compound of formula (I)

The compound according to the present invention can be in the form of a stereoisomer or a mixture of stereoisomers, such as a mixture of enantiomers or diastereoisomers, notably a racemic mixture. According to a specific embodiment, the compound of formula (I) is in the form of one diastereoisomer of configuration (S,S), of configuration (R,R), of configuration (S,R) or of configuration (R,S) as defined above.

The formula (I) as defined herein encompasses both active compounds, i.e drugs, and prodrugs thereof.

Preferably, the compound of formula (I) is in the form of a diastereoisomer of configuration (S,S) and responds to the following formula (I-A) :

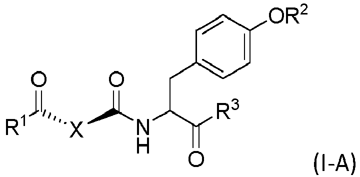

In a more preferred embodiment, the compound of formula (I-A) corresponds to the following enantiomer (I-A') :

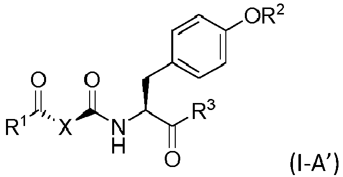

In a preferred embodiment, X is 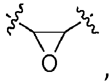 preferably 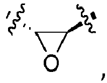 and R¹, R² and R³ are as defined in the present disclosure.

In another particular embodiment, X is 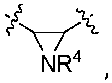 preferably 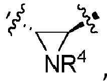 and R¹, R² and R³ are as defined in the present disclosure.

According to this embodiment, R⁴ is notably H or a C₁-C₁₂ aliphatic chain wherein up to 4 methylene units are optionally replaced by O, C(O), NH or N-C₁-C₆alkyl, said C₁-C₁₂ aliphatic chain being optionally substituted by one or more OH groups. In particular, R⁴ is selected in the group consisting of H, COOH, C(O)-C₁-C₆alkyl, (CH₂)ₚ-C(O)-C₁-C₆alkyl, C(O)-(CH₂)ₚ-C(O)-C₁-C₆alkyl, C(O)NHOH, (CH₂)ₚ-C(O)-NHOH and C(O)-(CH₂)ₚ-C(O)-NHOH, p being an integer from 1 to 4, said C₁-C₆ alkyl being notably methyl or ethyl, preferably ethyl.

In another particular embodiment, X is 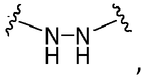 preferably 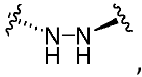 and R¹, R² and

R³ are as defined in the present disclosure.

According to some embodiments, R² is H, a C₁-C₆ aliphatic chain, such as C₁-C₆ alkyl, or C₁-C₆ alkyl-aryl, said aliphatic chain or alkyl-aryl being optionally substituted. Preferably, R² is H, optionally substituted C₁-C₆ alkyl, or C₁-C₆ alkyl-aryl, such as benzyl. When R² is a C₁-C₆ alkyl, such as methyl or ethyl, it is notably unsubstituted or substituted by one or more halogen, OH, NH₂, N(C₁-C₆alkyl)₂ or N⁺(C₁-C₆alkyl)₃, in particular N⁺(CH₃)₃. More preferably, R² is H, C₁-C₆ alkyl or benzyl.

According to some other embodiments, R³ is OH, O-C₁-C₆ aliphatic chain or NHOH, said aliphatic chain being optionally substituted. Preferably, R³ is OH, optionally substituted O-C₁-C₆ alkyl, O-C₁-C₆ alkenyl, or NHOH. When R³ is a O-C₁-C₆ alkyl, such as O-methyl or O-ethyl, said alkyl is notably unsubstituted or substituted by one or more halogen, OH, NH₂, NH-C₁-C₆alkyl, N(C₁-C₆alkyl)₂ or N⁺(C₁-C₆alkyl)₃, in particular N⁺(CH₃)₃. More preferably, R³ is OH or O-C₁-C₆ alkyl, in particular O-ethyl.

According to some embodiments, R¹ is O-C₁-C₆ alkyl, O-C₂-C₆ alkenyl or NH-OH, provided that when R³ is OH, R¹ is not O-C₁-C₆ alkyl. Preferably, R¹ is O-methyl, O-ethyl, O-allyl or NHOH, provided that when R³ is OH, R¹ is not O-C₁-C₆ alkyl. When R¹ is O-C₁-C₆ alkyl, R³ is preferably O-C₁-C₆ alkyl such as O-methyl or O-ethyl, unsubstituted or substituted by one or more halogen, OH, NH₂, NH-C₁-C₆alkyl, N(C₁-C₆alkyl)₂ or N⁺(C₁-C₆alkyl)₃, in particular N⁺(CH₃)₃. More preferably, when R¹ is O-C₁-C₆ alkyl, notably O-ethyl, R³ is O-ethyl.

According to a preferred embodiment, R¹ is 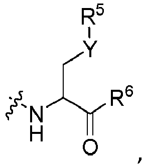 preferably

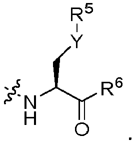

The compound of formula (I) may be a compound of the following formula : 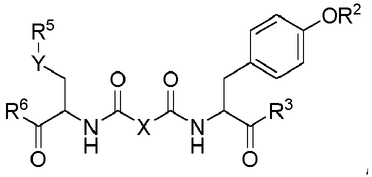 in which R², R³, R⁵, R⁶, X and Y are as defined in the present disclosure.

Preferably, the compound of formula (I) is a compound of formula (I-Aₐ) having the following configuration : 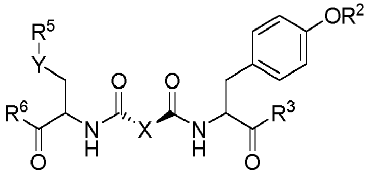 (I-Aₐ), in which R², R³, R⁵, R⁶, X and Y are as defined in the present disclosure.

More preferably, the compound of formula (I) is a compound of formula (I -A'ₐ) having the following configuration : 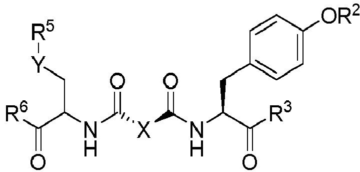 (I-A'a), in which R², R³, R⁵, R⁶, X and Y are as defined in the present disclosure. Y may notably be 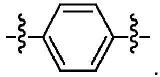 In such an embodiment, R⁵ is preferably OH, O-C₁-C₆ alkyl, O-aryl, O-heteroaryl, O-C₁-C₆ alkyl-aryl, O-C₁-C₆ alkyl-heteroaryl, C(O)OH, C(O)O-C₁-C₆ alkyl or C(O)NHOH, said alkyl, alkenyl, alkynyl, aryl, heteroaryl, alkyl-aryl or alkyl-heteroaryl being optionally substituted. More preferably, R⁵ is OH, O-C₁-C₆ alkyl, such as O-methyl or O-ethyl, or O-C₁-C₆ alkyl-aryl, such as O-benzyl.

Alternatively, Y may be ―(CH₂)ₘ―, with m being an integer from 0 to 6, preferably from 0 to 4. More preferably, m is 1 or 2, even more preferably m is 2. In the embodiment wherein Y is - (CH₂)ₘ, R⁵ is preferably C(O)OH, C(O)O-C₁-C₆ alkyl or C(O)NHOH, said alkyl being optionally substituted. More preferably, R⁵ is C(O)OH, C(O)OEt or C(O)NHOH, in particular COOH.

According to some embodiments, R⁶ is OH or O-C₁-C₆ alkyl, such as O-methyl or O-ethyl, notably O-ethyl, in particular when Y is

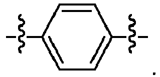

According to other specific embodiments, R⁶ is NH-CH(R⁷)-(CH₂)ₙ-R⁸, in particular when Y is - (CH₂)ₘ―, notably is ―(CH₂)₂―. In such embodiments, R⁷ is preferably H, C₁-C₆ alkyl, C₁-C₆ alkyl-aryl, such as benzyl or C₁-C₆ alkyl-heteroaryl, such as CH₂-indole group. R⁸ is preferably C(O)NH₂, aryl, such as phenyl, heteroaryl, such as indole, SH, or S-C₁-C₆alkyl, such as S-C(CH₃)₃ and n is preferably 0, 1, 2 or 3. In some embodiments, R⁷ is C₁-C₆ alkyl, R⁸ is C(O)NH₂ and n is 0. In some other preferred embodiments, R⁷ is H, R⁸ is phenyl and n is 1, 2 or 3, preferably 1.

According to a preferred embodiment, when R³ is OH, R¹ is 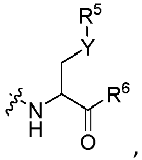 preferably

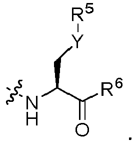

In a preferred embodiment, the compound of formula (I) is a compound of formula (1-A'a) as defined above, in which X, R² and R³ are as defined above, R¹ is 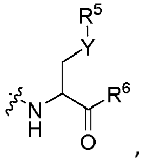 preferably 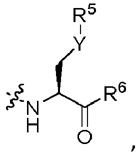 with:
- Y being ―(CH₂)ₘ―, m being as defined above and notably m is 2, R⁵ being C(O)OH, C(O)OEt or C(O)NHOH, R⁶ being NH-CH(R⁷)-(CH₂)ₙ-R⁸, R⁷ being H, C₁-C₆ alkyl, C₁-C₆ alkyl-aryl, such as benzyl or C₁-C₆ alkyl-heteroaryl, such as CH₂-indole group, R⁸ being C(O)NH₂, aryl, such as phenyl, heteroaryl, such as indole, SH, or S-C₁-C₆alkyl, such as S-C(CH₃)₃ and n being 0, 1, 2 or 3, or
- Y being 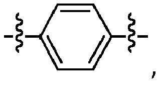 R⁵ being OH, O-C₁-C₆ alkyl, such as O-methyl or O-ethyl, O-C₁-C₆ alkyl-aryl, such as O-benzyl, and R⁶ being OH or O-C₁-C₆ alkyl, such as O-methyl or O-ethyl.

According to another preferred embodiment, the compound of formula (I) is of formula (I-A') in whichX is 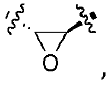 R¹ is O-C₁-C₆alkyl, in particular O-ethyl, R² is H or C₁-C₆ alkyl, in particular ethyl, and R³ is OH or O-C₁-C₆ alkyl, in particular O-ethyl.

In a more preferred embodiment, the compound of formula (I) is of formula (I-A'ₐ) in which X is 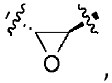 R² is H, C₁-C₆ alkyl, in particular ethyl, or benzyl, R³ is OH or O-C₁-C₆ alkyl, in particular O-ethyl, and R¹ is 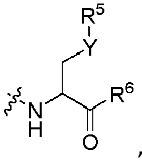 preferably 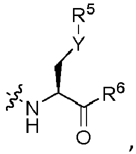 in which:
- Y is ―(CH₂)ₘ―, in particular ―(CH₂)₂-, R⁵ is C(O)NHOH, R⁶ is NH-CH₂-(CH₂)ₙ-R⁸, with R⁸ being aryl, such as phenyl, and n being 1, 2 or 3, notably 1, or
- Y is 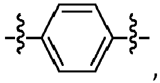 R⁵ and R⁶ are independently OH or O-C₁-C₆ alkyl, such as O-methyl or O-ethyl, in particular O-ethyl.

In particular, the compound of formula (I) is not :

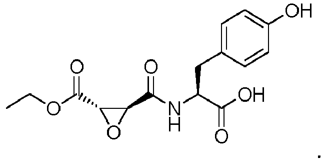

According to a particular embodiment, the compound of formula (I) is a peptide based-VASH inhibitor. By 'peptide based-VASH inhibitor' according to the invention, it means a VASH inhibitor containing a peptidic moiety constituted of 1 to 20 amino acids, wherein the most C-terminal amino acid is selected from Y or F, and able to target and inhibit, at least partially, the activity of proteins having a tubulin carboxypeptidase activity, and thereby inhibit microtubule detyrosination.

The term *"peptidic moiety"* refers to a moiety containing at least one amino acid and at most 20 amino acids. When the peptidic moiety comprises two or more amino acids, said amino acids are linked together by peptide bonds and chemically modified or not.

According to a preferred embodiment, the compound of formula (I) is selected among: 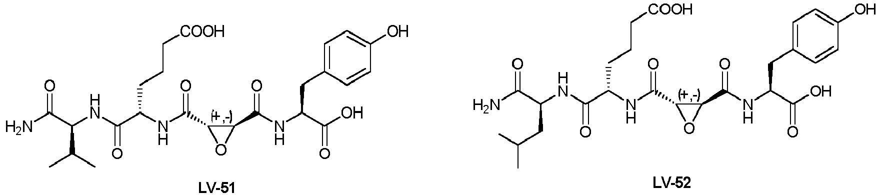 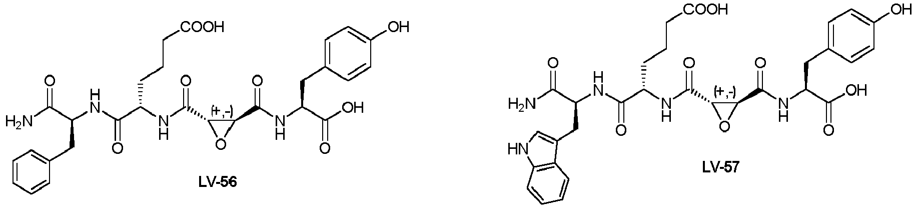 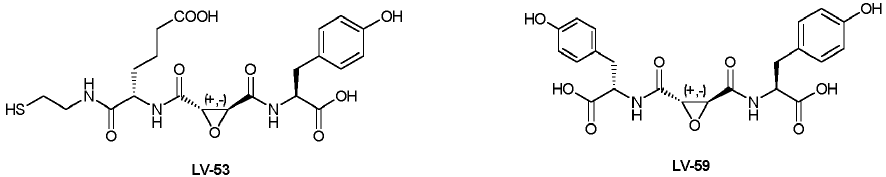 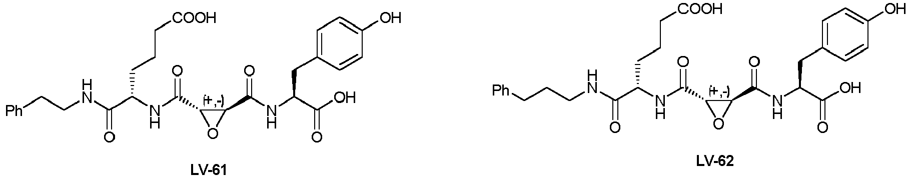 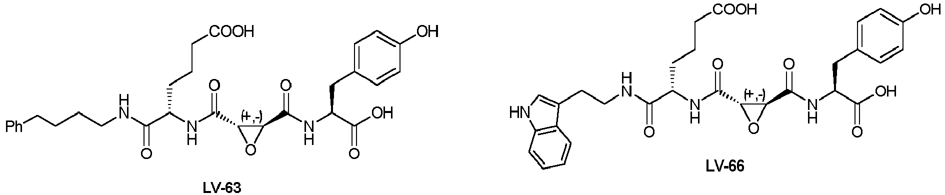 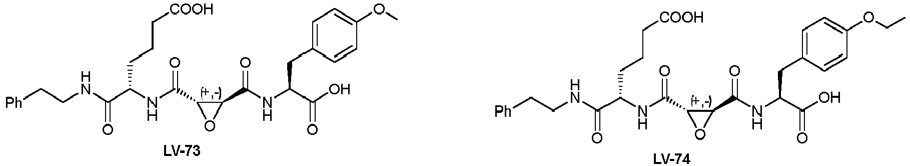 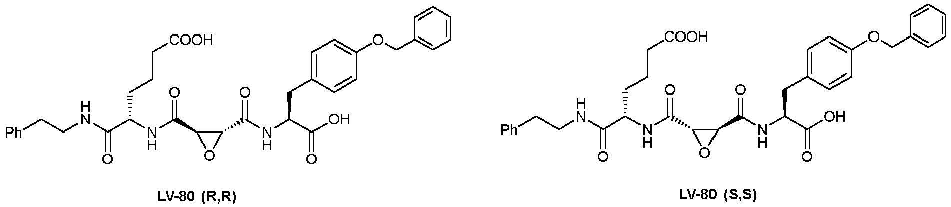 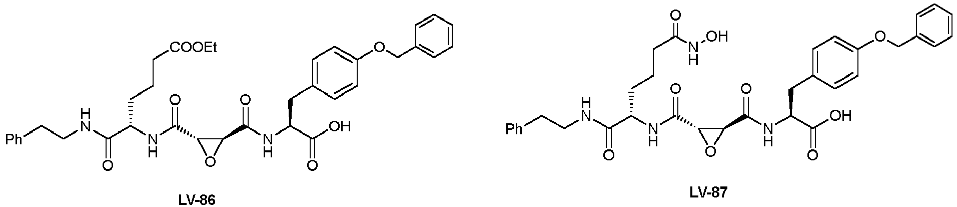 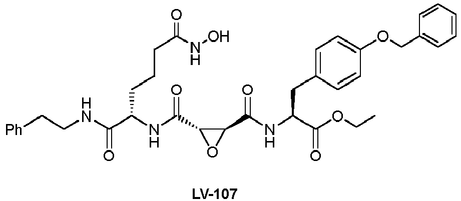 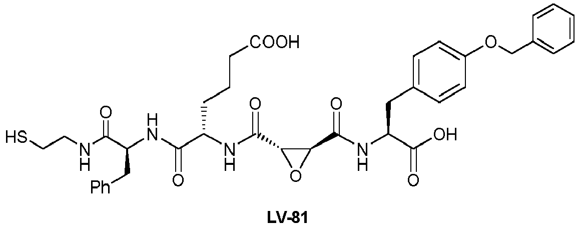 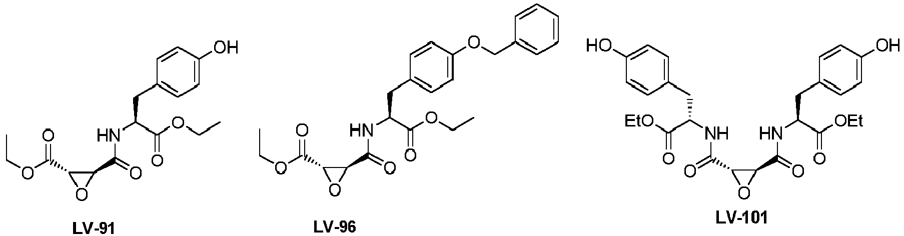 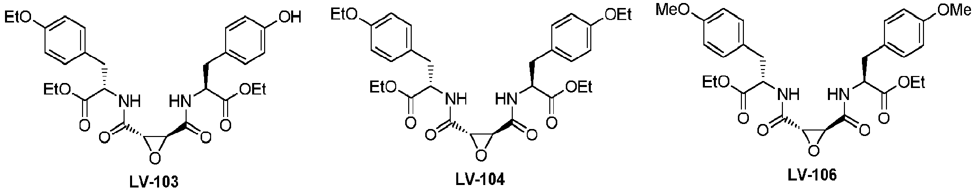 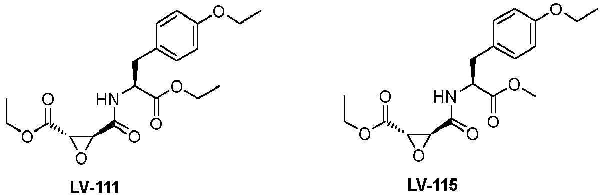 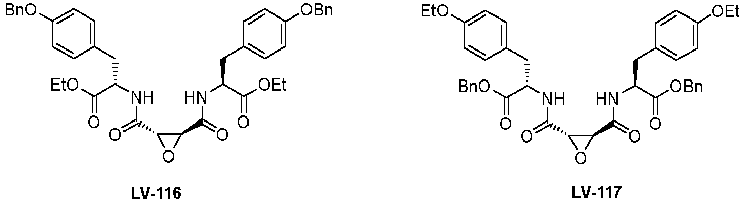 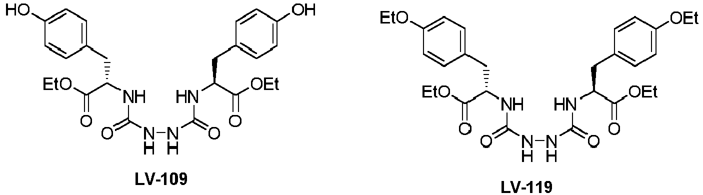 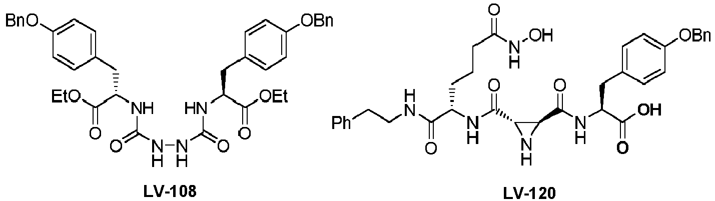 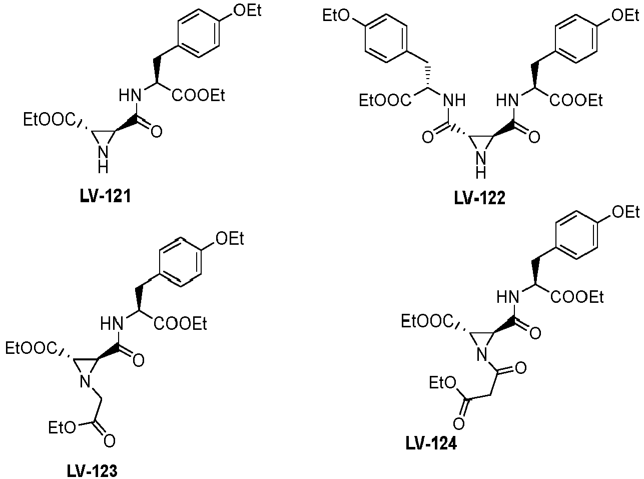 and the pharmaceutically acceptable salts and/or solvates thereof.

In a particular and preferred embodiment, the compound of formula (I) according to the invention is selected in the group consisting of compounds LV-80 to LV-124 or conjugates thereof.

In a particular embodiment, the compound of formula (I) according to the invention is the compound LV-80 or a conjugate thereof.

In a particular embodiment, the compound of formula (I) according to the invention is the compound LV-91 or a conjugate thereof.

In a particular embodiment, the compound of formula (I) according to the invention is the compound LV-104 or a conjugate thereof. LV-104 may be a prodrug of the corresponding active drug.

In a particular embodiment, the compound of formula (I) according to the invention is the compound LV-111 or a conjugate thereof. LV-111 may be a prodrug of the corresponding active drug.

### Method of preparation of a compound of formula (I)

The compound of formula (I) as described above, or a pharmaceutically acceptable salt and/or solvate thereof, may be obtained by a method comprising the following steps:
(a) reacting a compound of formula (II) : 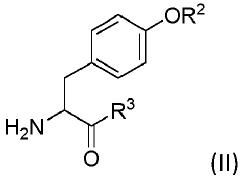 with a compound of formula (III) : 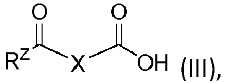 wherein R^{Z} is R¹ as defined above or OH,
(b) optionally, converting R^{Z} being OH to R¹ as defined above.

The compound of formula (III) may be obtained according to methods well-known from the skilled person in the art.

The reaction between compound of formula (II) and formula (III) is notably a peptide coupling as defined above.

Optionally, additional steps of protection/deprotection and/or of functionalization well-known from the skilled person in the art may occur before or after the reaction between compounds of formula (II) and (III) to afford compound of formula (I) with the suitable substituents as described above.

In particular, when R^{Z} is OH, the compound resulting from step (a) undergoes a peptide coupling with 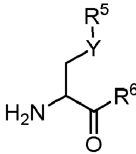 to provide a compound of formula (I) in which R¹ is

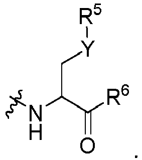

The one or more peptide coupling carried out in the method of preparation of compound of formula (I) are notably achieved in presence of PyAOP as coupling agent. Preferably, the base DIEA is also used.

The peptide coupling may be carried out on a solid support, notably by using a resin to which one of the reagents, the amine part or the acid part, is attached. Such methods are well-known from the skilled person in the art.

### Conjugate comprising a fragment of a compound of formula (I) linked to a biomolecule

The present invention also relates to a conjugate comprising a fragment of a compound of formula (I) as described above linked to a biomolecule such as a peptide, a protein, a biomarker, as for example a photolabelling agent, such as Rhodamine, cyanines derivatives or fluoresceine, an affinity probe such as biotin, or a E3 ubiquitin ligase recruiter including but not limited to Thalidomide, VH032, VH101, dBET1, dFKBP12, QCA570, PROTAC6, ZNL-02-096 or d9A-2.

According to a particular embodiment, the conjugate according to the present invention has the following formula (I'): 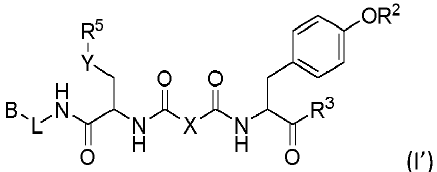 or a pharmaceutically acceptable salt and/or solvate thereof, wherein
B is a biomolecule such as a peptide, a protein, a biomarker, as for example a photolabelling agent, or a E3 ligase recruiter such as Thalidomide,
L is a linker, and
R², R³, X, Y and R⁵ are as defined above.

According to a preferred embodiment, the conjugate of formula (I') is a conjugate of formula (I'-A) having the following configuration:

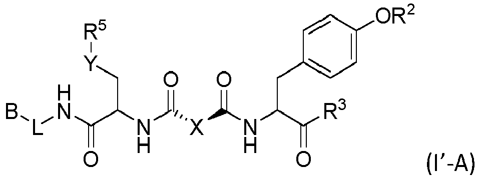

In particular, the conjugate of formula (I') is a conjugate of formula (I'-Aₐ) having the following configuration :

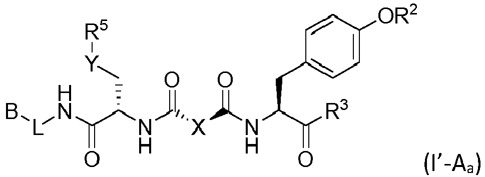

In the conjugate of formula (I'), in particular of formula (I'-Aa), X is preferably 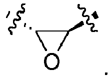 R² is advantageously H or C₁-C₆ alkyl, in particular ethyl, or benzyl. R³ is preferably OH or O-C₁-C₆ alkyl, in particular O-ethyl.

Y may be ―(CH₂)ₘ- or 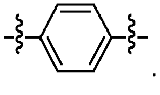 When Y is ―(CH₂)ₘ―, in particular ―(CH₂)₂-, R⁵ is preferably C(O)NHOH, R⁶ is preferably NH-CH₂-(CH₂)ₙ-R⁸, with R⁸ being advantageously aryl, such as phenyl, and n being advantageously 1, 2 or 3, notably 1. When Y is 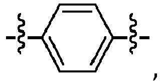 R⁵ is preferably OH or O-C₁-C₆ alkyl, such as O-methyl or O-ethyl, in particular O-ethyl.

According to some embodiments, the linker L corresponds to a divalent radical derived from a C₁-C₁₂ aliphatic chain, wherein one or more methylene unit(s) are replaced by structural linkers selected from arylene or fragments ―O―, ―S―, ―C(=O)―, ―SO₂― or ―N(C₁-C₆ alkyl)―, wherein said aliphatic chain is unsubstituted or is substituted by one or more radicals selected from halogen, OH, a C₁-C₆ alkyl and/or a C₁-C₆ alkyl aryl group, such as benzyl group.

Preferably, the biomolecule is E3 ligase recruiter, notably Thalidomide, and the conjugate is thus a PROTAC E3 ligase recruiter.

In a preferred embodiment, the conjugate is thus a PROTAC E3 ligase recruiter of the following formula (II') : 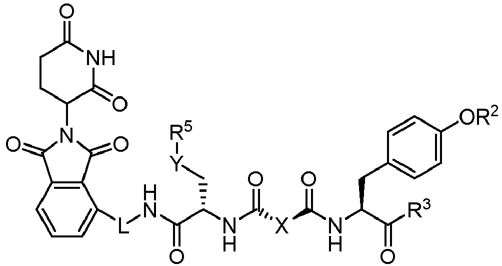 (II'), in which X, Y, L, R², R³ and R⁵ are as defined above.

For example, the PROTAC E3 ligase recruiter is 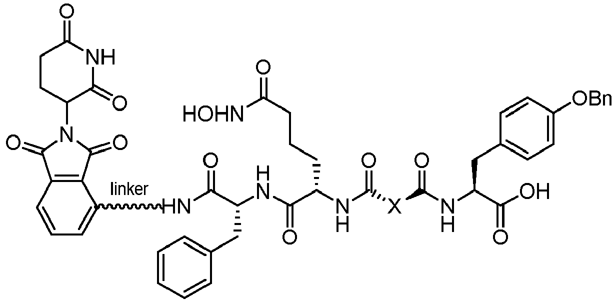 in which X is as defined above, in particular X is

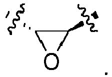

In another embodiment, the biomolecule is an affinity probe and the conjugate of formula (I') may be : 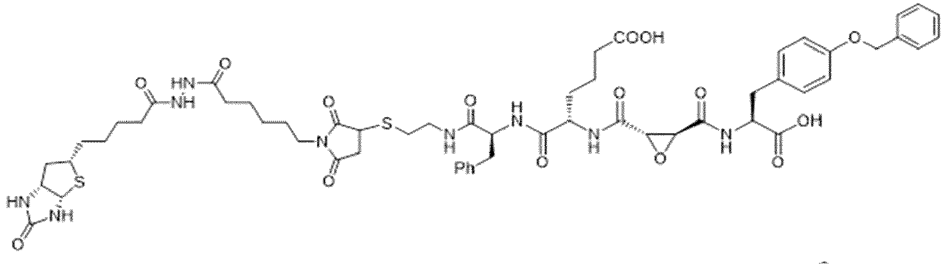 in which a compound of formula (I) is linked to biotin.

In another embodiment, the biomolecule is a photolabeling agent and the conjugate of formula (I') may be : 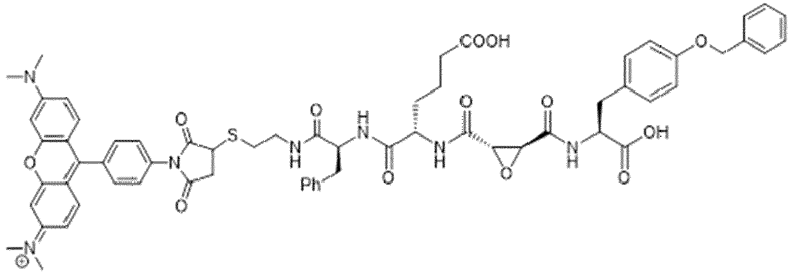 in which a compound of formula (I) is linked to Rhodamine.

### Pharmaceutical composition

The present invention also relates to a pharmaceutical composition comprising at least one pharmaceutically acceptable excipient and at least one compound of formula (I) as described above or a pharmaceutically acceptable salt and/or solvate thereof.

The present invention also relates to a pharmaceutical composition comprising at least one conjugate as described above, such as a conjugate of formula (I'), or a pharmaceutically acceptable salt and/or solvate thereof, and at least one pharmaceutically acceptable excipient.

The pharmaceutical compositions of the invention can be intended to oral or parenteral (including but not limited to subcutaneous, intramuscular, intravenous, ocular, intravitreal, topical, sublingual administration, preferably oral or intravenous administration. The active ingredient can be administered in unit forms for administration, mixed with conventional pharmaceutical carriers, to animals, preferably mammals including humans.

For oral administration, the pharmaceutical composition can be in a solid or liquid (solution or suspension) form.

A solid composition can be in the form of tablets, gelatin capsules, powders, granules and the like. In tablets, the active ingredient can be mixed with pharmaceutical vehicle(s) such as gelatin, starch, lactose, magnesium stearate, talc, gum arabic and the like before being compressed. The tablets may be further coated, notably with sucrose or with other suitable materials, or they may be treated in such a way that they have a prolonged or delayed activity. In powders or granules, the active ingredient can be mixed or granulated with dispersing agents, wetting agents or suspending agents and with flavor correctors or sweeteners. In gelatin capsules, the active ingredient can be introduced into soft or hard gelatin capsules in the form of a powder or granules such as mentioned previously or in the form of a liquid composition such as mentioned below.

A liquid composition can contain the active ingredient together with a sweetener, a taste enhancer or a suitable coloring agent in a solvent such as water. The liquid composition can also be obtained by suspending or dissolving a powder or granules, as mentioned above, in a liquid such as water, juice, milk, etc. It can be for example a syrup or an elixir.

For parenteral administration, the composition can be in the form of an aqueous suspension or solution which may contain suspending agents and/or wetting agents. The composition is advantageously sterile. It can be in the form of an isotonic solution (in particular in comparison to blood).

### Uses

### Therapeutic uses

The compound of formula (I), the pharmaceutically acceptable salt and/or solvate thereof, or the pharmaceutical composition according to the invention act as VASH inhibitors, meaning that it is able to inhibit the peptidase activity of VASH that catalyzes microtubules detyrosination. When this VASH peptidase activity is deregulated, and notably abnormally increased, it induces severe disorders as defined below.

The compounds of formula (I) in which X is 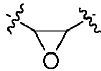 or 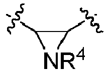 are notably irreversible VASH inhibitors, which covalently bind to the VASH enzymes.

The compounds of formula (I) in which X is 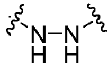 are notably reversible VASH inhibitors, meaning that VASH peptidase enzymatic activity may be recovered.

The present invention relates to a compound of formula (I) according to the invention or a pharmaceutically acceptable salt and/or solvate thereof, for use as a drug, in particular in the prevention and/or the treatment of a VASH peptidase activity associated disorder.

In other terms, the present invention relates to the use of a compound of formula (I) according to the invention or a pharmaceutically acceptable salt and/or solvate thereof, for the manufacture of a drug, notably intended in the prevention and/or the treatment of a VASH peptidase activity associated disorder.

In other terms, the present invention relates to the use of a compound of formula (I) according to the invention or a pharmaceutically acceptable salt and/or solvate thereof for the prevention and/or the treatment of a VASH peptidase activity associated disorder.

In other terms, the present invention relates to a method for the prevention and/or the treatment of a VASH peptidase activity associated disorder comprising the administration to a person in need thereof of an effective dose of a compound of formula (I) according to the invention or a pharmaceutically acceptable salt and/or solvate thereof.

According to another aspect, the present invention relates to a pharmaceutical composition according to the invention for use as a drug, in particular in the prevention and/or the treatment of a VASH peptidase activity associated disorder.

In other terms, the present invention relates to the use of a pharmaceutical composition according to the invention for the manufacture of a drug, notably intended in the prevention and/or the treatment of a VASH peptidase activity associated disorder.

In other terms, the present invention relates to the use of a pharmaceutical composition according to the invention for the prevention and/or the treatment of a VASH peptidase activity associated disorder.

In other terms, the present invention relates to a method for the prevention and/or the treatment of a VASH peptidase activity associated disorder comprising the administration to a person in need thereof of an effective dose of a pharmaceutical composition according to the invention.

According to another aspect, the present invention relates to a conjugate according to the invention for use as a drug, in particular in the prevention and/or the treatment of a VASH peptidase activity associated disorder.

In other terms, the present invention relates to the use of a conjugate according to the invention for the manufacture of a drug, notably intended in the prevention and/or the treatment of a VASH peptidase activity associated disorder.

In other terms, the present invention relates to the use of a conjugate according to the invention for the prevention and/or the treatment of a VASH peptidase activity associated disorder.

In other terms, the present invention relates to a method for the prevention and/or the treatment of a VASH peptidase activity associated disorder comprising the administration to a person in need thereof of an effective dose of a conjugate according to the invention.

A preferred embodiment relates to a PROTAC E3 ligase recruiter according to the present invention, notably of formula (I") as defined above, for use as a drug, in particular in the prevention and/or the treatment of a VASH peptidase activity associated disorder. Such a PROTAC E3 ligase recruiter is able to selectively target VASH enzyme and to induce its total degradation by proteasome.

The conjugate of formula (I") in which X is 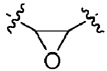 or 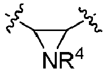 is notably an irreversible PROTAC E3 ligase recruiter.

The conjugate of formula (I") in which X is 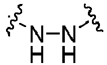 is notably reversible PROTAC E3 ligase recruiter.

According to the present invention, a VASH peptidase activity-associated disorder is preferably selected from fibrosis, cancer and a tubulin carboxypeptidase associated disease.

In particular, the VASH peptidase activity associated disorder is a disorder involving altered microtubule detyrosination and/or polyglutamylation, notably selected from neurodegenerative diseases, such as Alzheimer disease or Parkinson disease, glaucoma, psychiatric disorders, neuronal disorders, cancers, such as colon cancer and neuroblastoma, muscular dystrophies, infertility, retinal degeneration, Purkinje cell sicknesses, infantile onset degeneration, male infertilities and ciliopathies, in particular neurodegenerative diseases, cancers and muscular dystrophies.

In particular, a VASH peptidase activity-associated disorder is a tubulin carboxypeptidase associated disease including, without being limited to, disorder involving altered microtubule detyrosination.

According to a particular embodiment, a disorder involving altered microtubule detyrosination may be selected from neurodegenerative diseases, such as Alzheimer disease or Parkinson disease, glaucoma, psychiatric disorders, and neuronal disorders, cancers, such as colon cancer and neuroblastoma, muscular dystrophies, infertility, retinal degeneration and ciliopathies, in particular neurodegenerative diseases, cancers and muscular dystrophies.

In another aspect, a VASH peptidase activity-associated disorder is a tubulin carboxypeptidase associated disease including, disorder involving polyglutamination.

Accordingto a particular embodiment, a disorder involving polyglutamination may be selected from neurodegeneration, neurodevelopmental disorders, Purkinje cell sicknesses, infantile onset degeneration, ciliopathies, male infertilities, cancer, respiratory disorder, retinal degeneration, bleeding disorders, non-Mendelian inheretence disorders.

### Research tools uses

According to another aspect, the present invention relates to a compound of formula (I) according to the invention or a conjugate thereof for use as research tool for research and development activities, in particular selected in the group consisting of:
- *in vitro* and/or *in cellulo* screening assays for identifying new VASH inhibitors and/or quantifying their inhibitor efficiency,
- *in vitro* method for studying the role of tubulin detyrosination, in particular in VASH peptidase activity associated disorder, such as its occurrence, aggressiveness and progression,
- *in vitro* diagnostic methods for identifying or monitoring a VASH peptidase activity associated disorder involving altered microtubule detyrosination and/or polyglutamylation,
- and related kits for performing said screening assays and methods.

These R&D activities as examples of illustrative but not limitative uses of said compounds for formula (I) or conjugate thereof.

The said compounds for formula (I) or conjugate thereof is used in particular as positive control in said in vitro or in cellulo screening assays or methods.

The said compounds for formula (I) may be coupled for example but not limited to fluorescent dyes, UV-sensitive dyes, HRP, alkaline phosphatase, biotin.

In a particular embodiment, a compound of formula (I) or a conjugate thereof according to the invention is used in an *in vitro* screening assay of VASH inhibitors (primary screening assay), wherein the compound of formula (I) is used as positive control.

As an example, the screening assay is an immuno-assay wherein the compound of formula (I) or a conjugate thereof is coated on plates as positive control.

The invention also concerns an *in vitro* kit assay for a primary screening assay of VASH inhibitors comprising a compound of formula (I) or a conjugate thereof as positive control.

In another particular embodiment, a compound of formula (I) or a conjugate thereof according to the invention is used in an *in cellulo* screening assay of VASH inhibitors (secundary screening assay), wherein the compound of formula (I) or a conjugate thereof is used as positive control.

The invention also concerns an *in cellulo* kit assay for a secondary screening assay of VASH inhibitors comprising a compound of formula (I) or a conjugate thereof as positive control.

In a preferred embodiment, the *in cellulo* kit assay comprises CHL cell lines and a compound of formula (I) as positive control.

In another particular embodiment, a compound of formula (I) or a conjugate thereof according to the invention is used as tool to better understand the role of tubulin detyrosination, in particular in tubulin detyrosination associated disorder, such as its occurrence, aggressiveness and progression (for example cancer).

So the invention also concerns an *in vitro* screening assay for identification of VASH inhibitors comprising the steps of:
(i) Contacting (a) a substrate of VASH1 or VASH2 enzyme comprising an amino acids sequence having at least the last 4 amino acids residues of the C-terminal sequence of an α-tubulin and/or a Microtubule Associated Protein (MAP) and, as ultimate C-terminal amino acid residue, a tyrosine (Y), and b) an isolated or recombinant VAHS1 or VASH2; in the presence or absence (negative control) of the compound to be tested or in the presence of a compound of formula (I) or conjugate thereof according to the invention (positive control), and under conditions for cleavage and liberation of C-terminal free tyrosine (detyrosination);
(ii) Using reagents for detecting and measuring the signal related to cleavage and liberation of the C-terminal free tyrosine ;
(iii) Measuring and comparing the level of C-terminal free tyrosine, in presence and in absence (negative control) of the compound to be tested and in presence of the compound of formula (I) or conjugate thereof according to the invention (positive control), and
(iv) Selecting the compounds for which the level of C-terminal free tyrosine, is decreased in the presence of the compound to be tested (VASH inhibitors),
(v) Optionally classifying the inhibitor efficiency of the said VASH inhibitors compounds in comparison to the level of C-terminal free tyrosine in the presence of the compound of formula (I) or conjugate thereof according to the invention (positive control).

In a particular embodiment, the substrate of VASH1 or VASH2 enzyme is selected in the group consisting of purified and recombinant alpha-tubulin and recombinant engineered telokin as disclosed in the application WO2020/012002.

In a particular embodiment, the step (ii) of detection of the C-terminal free tyrosine uses a colorimetric assay using a tyrosinase.

In another particular embodiment, the *in vitro* screening assay is an immuno-assay, in which the step (ii) comprises :
- adding an effective amount of specific labelled antibody raised against detyrosinated alpha-tubulin (dTyr-Ab), under conditions that favor the formation of a complex antibody - cleaved substrate; and
- means for revealing the labelled signal;
or alternatively
- adding an effective amount of a primary specific antibody raised against detyrosinated alpha-tubulin (dTyr-Ab), under conditions that favor the formation of a complex antibody - cleaved substrate;
- adding an effective amount of a secondary labelled antibody specific of the primary antibody, under conditions that favor the formation of a complex primary antibody- cleaved substrate- labelled secondary antibody, and
- means for revealing the labelled signal,
and wherein the reaction occurs in soluble (fluid) phase or solid phase.

In particular, the antibody is labelled with a marker selected in the group consisting of an enzyme, a fluorescent compound or fluorophore, a (chemo)luminescent compound or a radioactive element, preferably an enzyme and more preferably a peroxidase.

In particular, the immunoassay is an enzyme immunoassay, a fluoroimmunoassay, a luminescent immunoassay or a radioimmunoassay, preferably a dot-blot or an enzyme immunoassay (ELISA).

In a particular embodiment, the *in vitro* screening immunoassay for identification of VASH inhibitors occurs in solid phase, wherein:
- substrate of VASH1 or VASH2 is coated on solid support, in particular on membrane or microplate, and the VASH1 or VASH2 enzyme, the compound(s) to be tested and the antibodies raised against cleaved substrate, in particular detyrosinated α-tubulin (dTyr-Ab), optionally labelled or combined with labelled secondary antibodies raised against primary antibodies are added in the reaction solution, or
- alternatively, the antibodies raised against cleaved substrate, in particular detyrosinated α-tubulin (dTyr-Ab), are coated on solid support, in particular on membrane or microplate, and VASH1 or VASH2 enzyme, substrate of said VASH1 or VASH2, the compound(s) to be tested, and secondary labelled antibodies raised against cleaved substrate, are added in the reaction solution, or
- alternatively, the VASH1 or VASH2 enzyme is coated on solid support, in particular on membrane or microplate, and the substrate of said VASH1 or VASH2, the compound(s) to be tested, and the antibodies raised against cleaved substrate, in particular detyrosinated α-tubulin (dTyr-Ab), optionally labelled or combined with labelled secondary antibodies raised against primary antibodies, are added in the reaction solution.

The present invention also concerns a kit for performing the *in vitro* screening immuno-assay for identification of VASH inhibitors as disclosed above, comprising:
(i) a VASH1 or VASH2 enzyme substrate, preferably a recombinant alpha-tubulin or an engineered telokin as disclosed in the application WO2020/012002,
(ii) a VASH1 or VASH2 enzyme, preferably an isolated or recombinant VASH1 or VAHS2,
(iii) antibodies raised against cleaved substrate, preferably detyrosinated α-tubulin (dTyr-Ab), in particular raised against detyrosinated α-tubulin SF9 (dTyr-Ab SF9) and coupled to horseradish peroxidase (HRP), and optionally secondary antibodies raised against the cleaved substrate antibodies, preferably the detyrosinated α-tubulin antibodies,
(iv) a negative control comprising a non-tyrosinated isolated or recombinant alpha-tubulin or telokin,
(v) a compound of formula (I) or a conjugate thereof according to the invention as positive control,
(vi) a fluid vessel or alternatively a solid support for coating or pre-coating either the substrate of VASH1 or VASH2 either the VASH1 or VASH2 enzyme, preferably microplates,
(vii) reagents for allowing contact of said substrate with VASH1 or VASH2 enzyme in reaction conditions for substrate cleavage, preferably detyrosination,
(viii) reagents for detecting and measuring the level of substrate cleavage, preferably detyrosination ; and
(ix) optionally a notice of use.

In another particular embodiment, a compound of formula (I) or a conjugate thereof according to the invention is used in an *in cellulo* method for quantifying VASH peptidase activity.

The present invention also concerns an *in cellulo* screening assay for identification of VASH inhibitors comprising:
(i) culturing CHL-1 cells and plating them, in particular in multi-wells culture dish,
(ii) treating CHL-1 cells with taxol, in absence (negative control) or in presence of the putative VASH inhibitors to be tested or in presence of the compound for formula (I) or a conjugate thereof according to the present invention (positive control);
(iii) Detection of detyrosination activity contained in protein samples using specific labelled antibodies raised against detyrosinated alpha-tubulin (dTyr-Ab), under conditions that favor the formation of a complex antibody - cleaved substrate, and means for revealing the labelled signal,
(iv) measuring and comparing the level of taxol induced detyrosination, in presence and in absence (negative control) of the compound to be tested and in presence of the compound of formula (I) or conjugate thereof according to the invention (positive control),
(v) Selecting the compounds for which the level taxol induced detyrosination, is reduced in the presence of the compound to be tested (VASH inhibitors),
(vi) Optionally classifying the inhibitor efficiency of the said VASH inhibitors compounds in comparison to the reduced level of taxol induced detyrosination in the presence of the compound of formula (I) or conjugate thereof according to the invention (positive control).

The present invention also concerns a kit for performing the *in cellulo* screening assay for identification of VASH inhibitors comprising:
(i) CHL-1 cells
(ii) Taxol,
(iii) antibodies raised against detyrosinated α-tubulin (dTyr-Ab),
(iv) a compound of formula (I) or a conjugate thereof according to the invention as positive control,
(v) a fluid vessel or alternatively a solid support for coating or pre-coating the CHL-1 cells, preferably microplates,
(vi) reagents for allowing contact of said CHL-1 cells pre-treated with taxol with compounds to be tested or the positive control in reaction conditions for detyrosination,
(vii) reagents for detecting and measuring the reduced level of taxol induced detyrosination ; and
(viii) optionally a notice of use.

In another particular embodiment, a compound of formula (I) or a conjugate thereof according to the invention is used in an *in vitro* diagnostic method for detecting a VASH peptidase activity associated disorder.

So the invention also concerns a kit assay for *in vitro* diagnostic method, wherein the compound of formula (I) or a conjugate thereof is used as positive control.

### Description of the figures

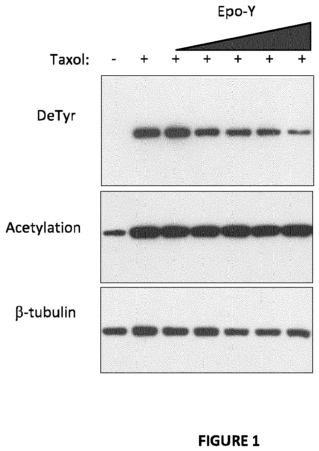

*Figure 1**:* In cellulo VASH-mediated tubulin detyrosination assay
   Effect of increasing concentrations of Epo-Y on tubulin detyrosination in human CHL-1 cells (melanoma derived cell line).

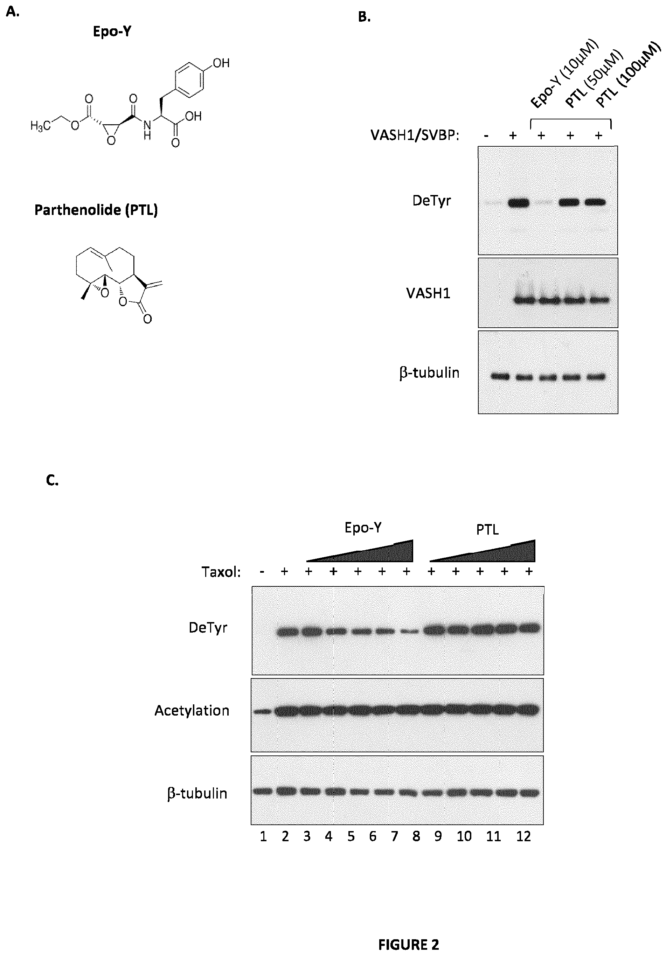

*Figure 2**:* Putative inhibitor parthenolide does not inhibit VASH-mediated tubulin detyrosination. (A) Chemical structure of Epo-Y and of the putative detyrosinase inhibitor parthenolide (PTL). (B) *In vitro* detyrosination assay using recombinant VASH1 proteins. DeTyr, detyrosination. (C) Effect of increasing concentrations of Epo-Y and PTL on tubulin detyrosination in human CHL-1 cells.

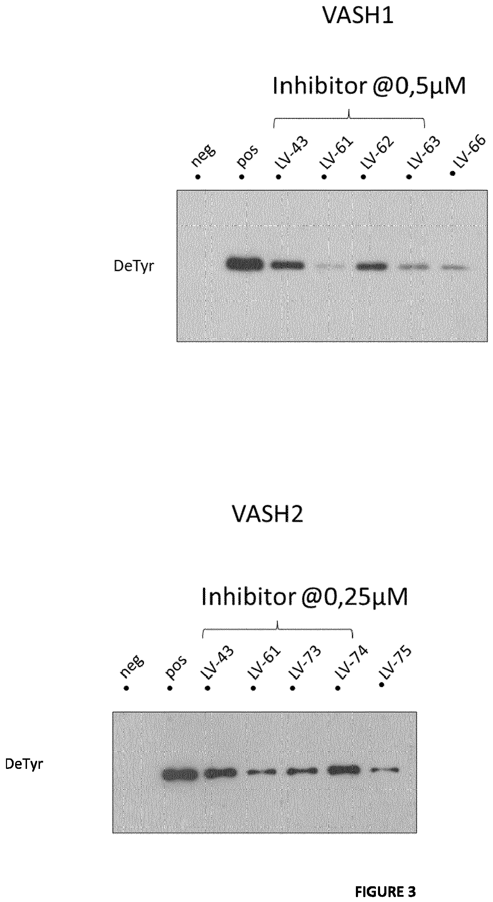

*Figure 3**:* newly developed inhibitors target VASH1 and VASH2 and peptidase activity VASH1 and VASH2-dependent tubulin detyrosination activities are both inhibited by newly designed inhibitors using the *in vitro* detyrosination assay.

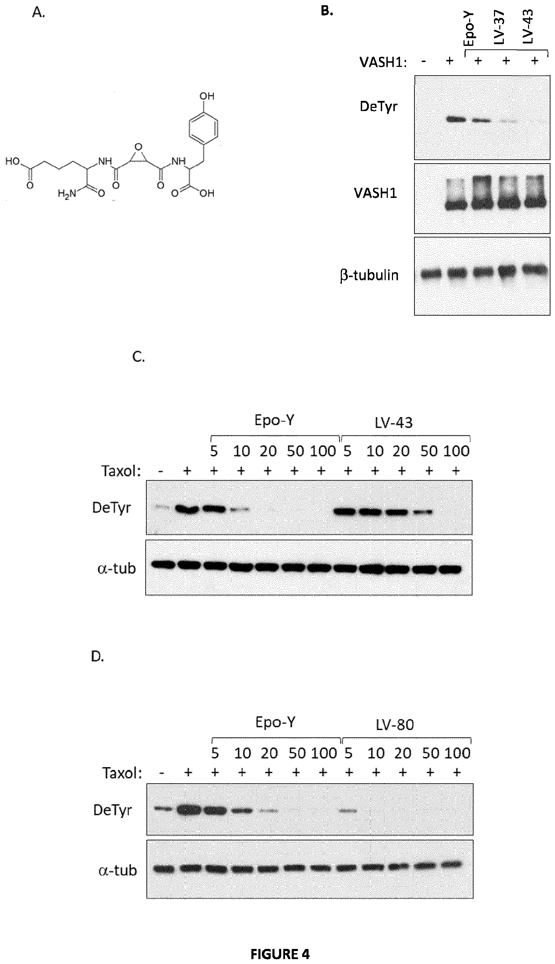

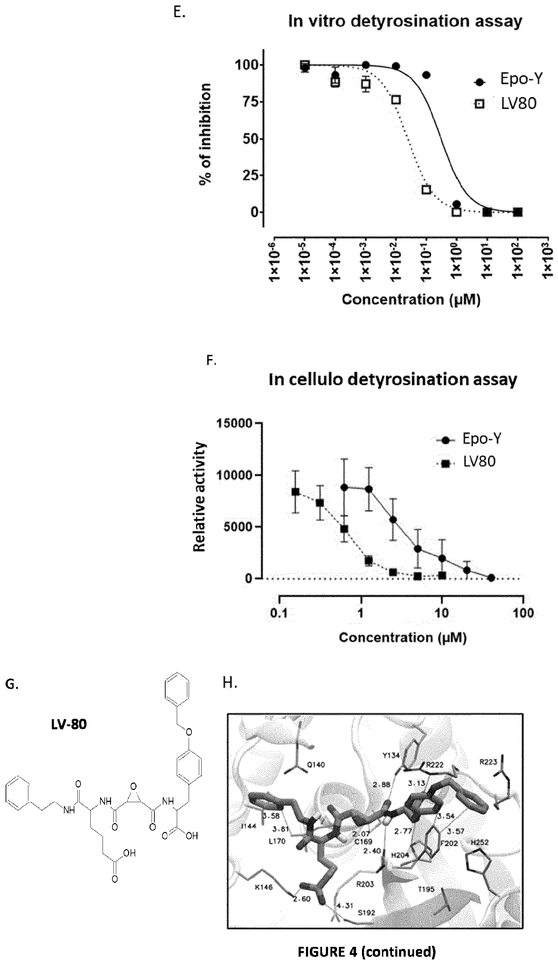

*Figure 4**:* Cell penetrant potent inhibitors of VASH peptidase activity (A) Chemical structure of LV-43 (outside the invention). (B) *In vitro* detyrosination assay using recombinant VASH1 proteins. (C) *In cellulo* detyrosination assay using recombinant VASH1 proteins. (D) *In cellulo* detyrosination assay using CHL-1 cells.(E) Comparison of the doseresponse curves obtained with Epo-Y and LV-80 *in vitro.* (F) Quantification of the effect of increasing concentrations of Epo-Y and LV-80 on tubulin detyrosination in human CHL-1 cells. (G) Chemical structure of LV-80 and (H) molecular docking to VASH1 displaying the putative binding mode (hydrogen bonds).

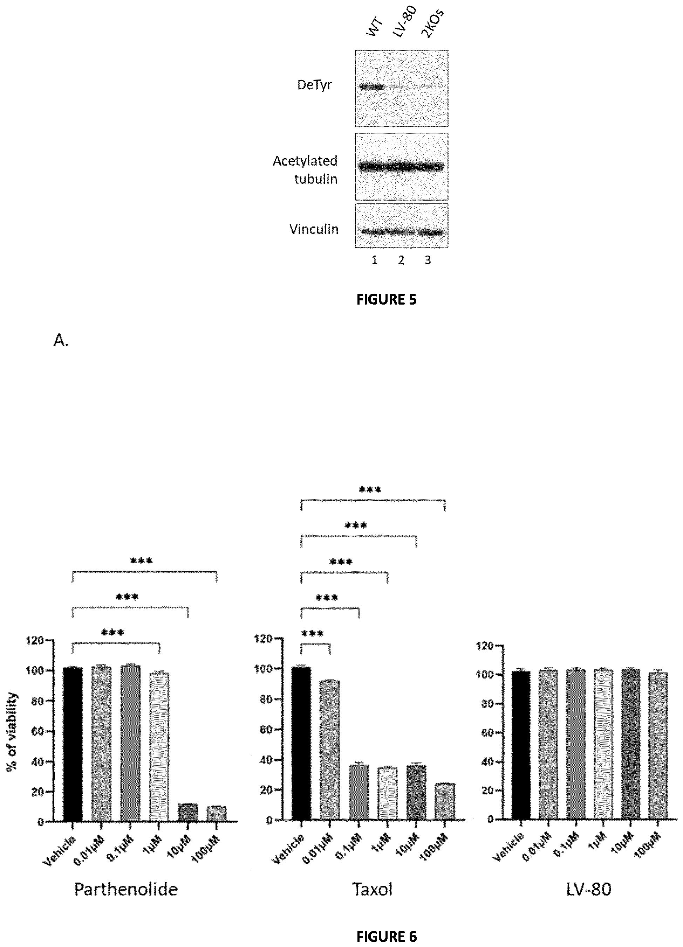

*Figure 5**:* Complete inhibition of VASH-mediated tubulin detyrosination in cells Analysis of the tubulin detyrosination levels in human CHL-1 cells incubated with LV-80 or vehicle (DMSO) for 24 hours and comparison with VASH1/2 knockout cells (2KOs) (disclosed in Nieuwenhuis J. et al. Science, 2017, 358(6369):1453-1456). Vinculin, loading control. DeTyr, detyrosination.

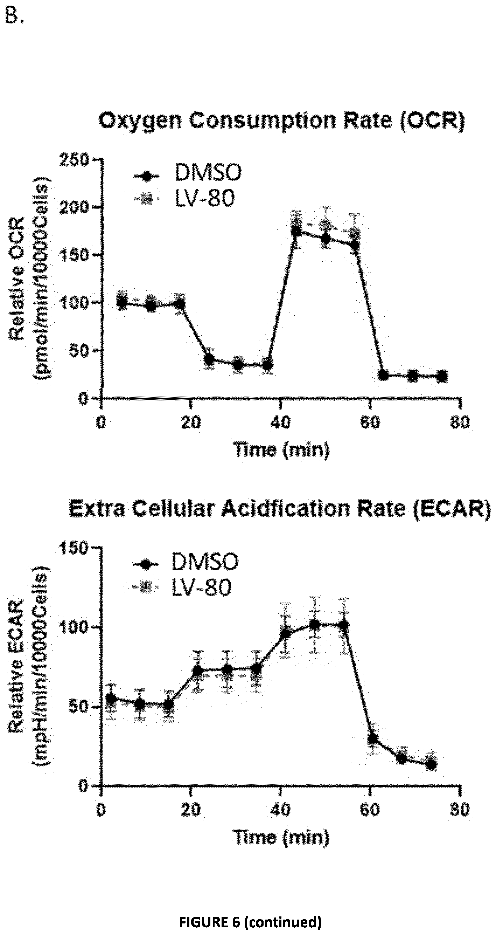

*Figure 6**:* Advantageous safety profile of VASH enzyme inhibitors (A) Cell viability studies using taxol (paclitaxel) and parthenolide as reference. (B) Measurement of cellular metabolism and extracellular acidification rate (ECAR) after incubation of CHL-1 cells with vehicle (DMSO) or the VASH-specific inhibitor LV-80 for 24 hours. Analysis of the oxygen consumption rate (OCR) and extracellular acidification rate (ECAR of live CHL-1 cells using the Seahorse XF Analyzer after 24 hours treatment. OCR rate is a key indicator of mitochondrial respiration and provides a systems-level view of cellular metabolic function in cultured cells.

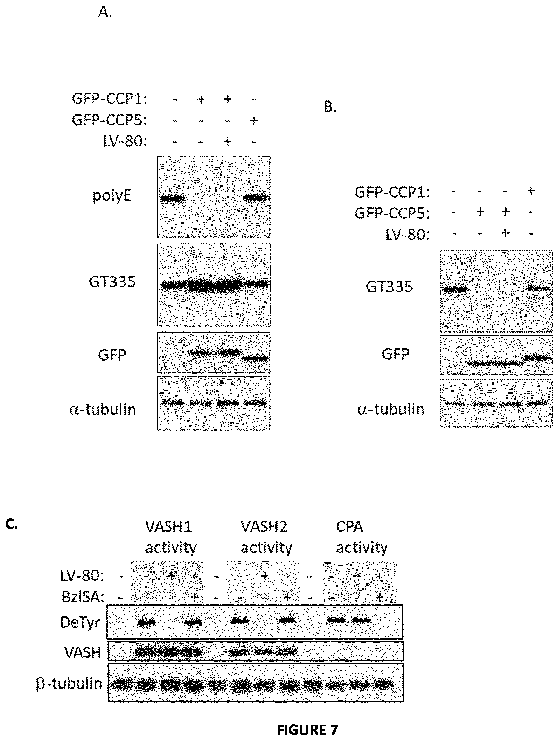

*Figure 7**:* Newly developed VASH inhibitors are specific to VASH enzymatic activity (A, B) Analysis of cytosolic carboxypeptidase 1 (CCP1) (A) and CCP5 (B) -mediated tubulin deglutamylation in the absence or presence of the VASH inhibitor LV-80 (50 µM). PolyE, antibody that recognizes long glutamate chains; GT335, antibody against branching point glutamate residues. (C) Specificity of LV-80. BzlSA, peptidomimetic benzylsuccinic acid.

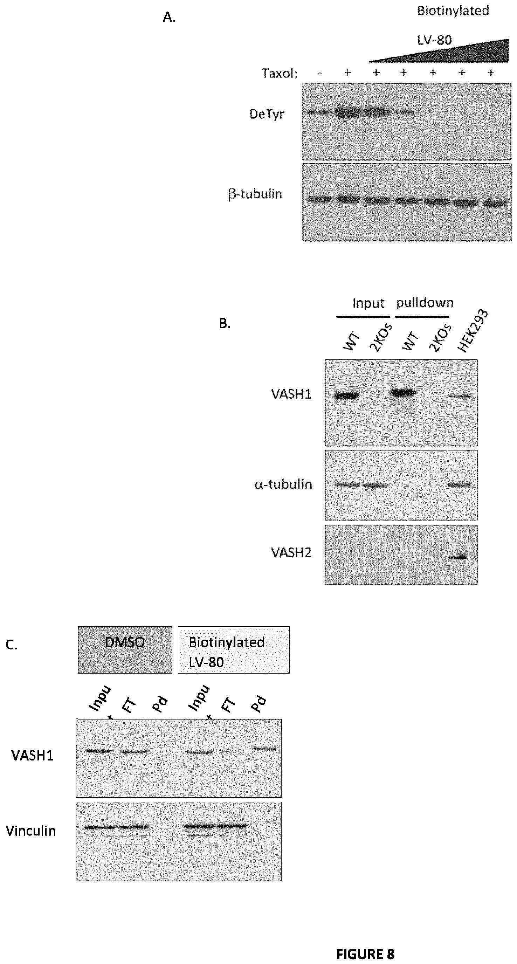

*Figure* 8: Biotinylated version of newly developed inhibitor as research tool (A) Effect of increasing concentrations of biotinylated LV-80 on tubulin detyrosination in human CHL-1 cells.(B) Pull-down assays of endogenous VASH using biotinylated LV-80. (C) Pull-down assays of endogenous VASH from human brain protein lysates using biotinylated LV-80.

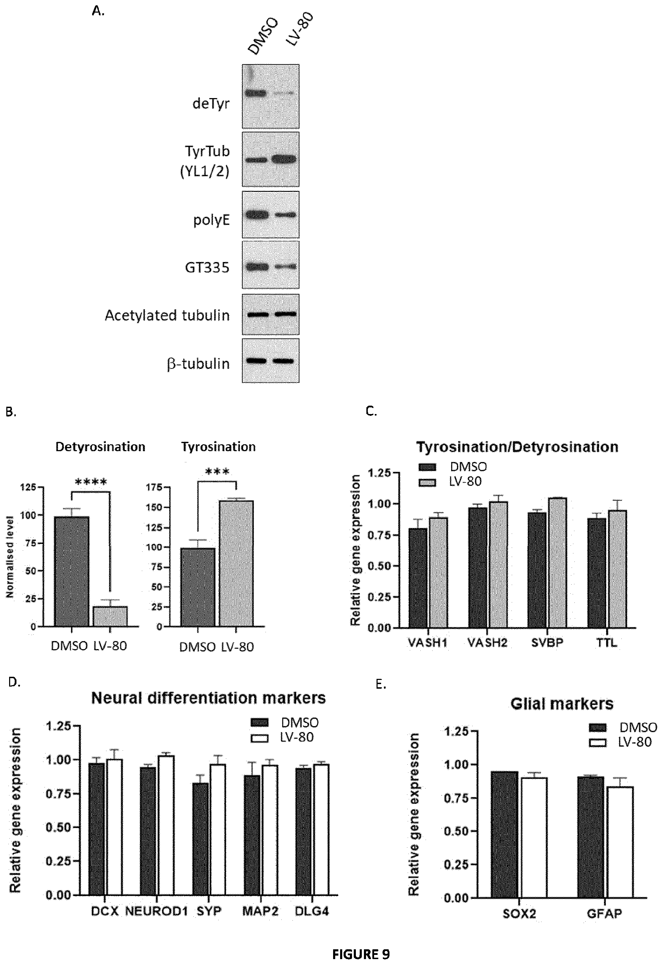

*Figure 9**:* Strong reduction of tubulin detyrosination in primary cortical neurons treated with VASH inhibitor. (A) Analysis of lysates of mouse primary cortical neurons incubated with vehicle (DMSO) or the VASH specific inhibitor LV-80. DeTyr, detyrosinated tubulin; TyrTub, tyrosinated tubulin; PolyE, antibody that recognizes long glutamate chains; GT335, antibody against branching point glutamate residues. (B) Quantification of tubulin detyrosination and tyrosination levels. P-values were calculated with the multiple t-test (n = 3, ^{∗}< 0.05, ^{∗∗}<0.01, ^{∗∗∗}<0.001, ^{∗∗∗∗}<0.0001). (C) Transcript levels of genes relevant to the detyrosination status analysed by qPCR. (D) Transcript levels of neural differentiation markers after incubation with vehicle (DMSO) or inhibitor. (E) Transcript levels of glial markers after incubation with vehicle (DMSO) or inhibitor for 7 days.

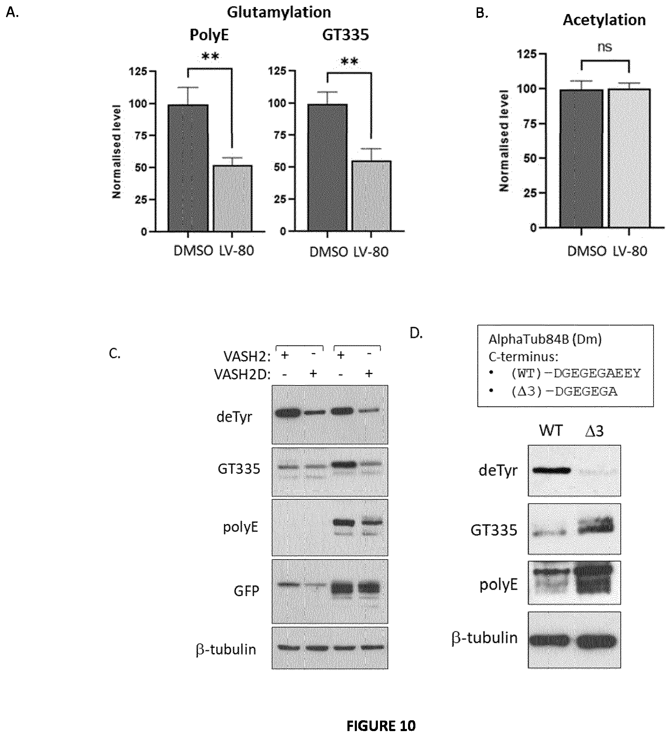

*Figure* 10: Detyrosination renders tubulin permissive to glutamylation (A) tubulin glutamylation levels, (B) and tubulin acetylation levels in mouse primary cortical neurons that were incubated with vehicle (DMSO) or the VASH specific inhibitor LV-80. P-values were calculated with the multiple t-test (n = 3, ^{∗}< 0.05, ^{∗∗}<0.01, ^{***}<0.001, ^{****}<0.0001). (C) Cross-talk between tubulin detyrosination and glutamylation. (D) Analysis of total Drosophila protein extracts.

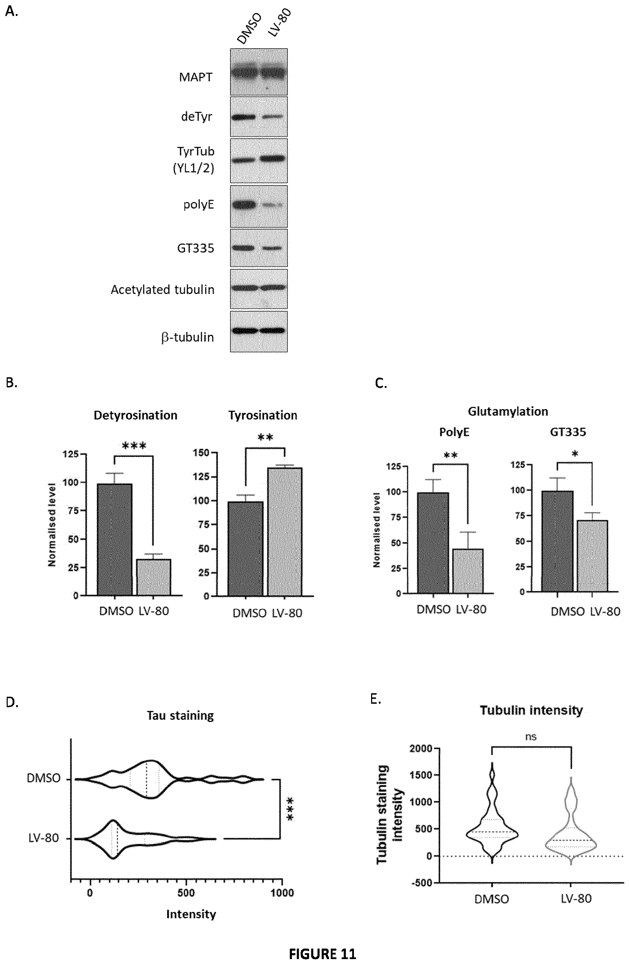

*Figure 11**:* Tubulin detyrosination impacts tau protein binding in primary cortical neurons (A) Analysis of mouse primary cortical neurons incubated with vehicle (DMSO) or the VASH specific inhibitor LV-80 for 3 days after 5 days of differentiation. DeTyr, detyrosinated tubulin; TyrTub, tyrosinated tubulin; PolyE, antibody that recognizes long glutamate chains; GT335, antibody against branching point glutamate residues. (B, C) Quantification of tubulin detyrosination and tyrosination levels (B), and tubulin glutamylation levels (C) in mouse primary cortical neurons incubated with vehicle (DMSO) or the VASH specific inhibitor LV-80 for 3 days after 5 days of differentiation as in (A). P-values were calculated with the multiple t-test (n = 3, ^{∗}< 0.05, ^{∗∗}<0.01, ^{∗∗∗}<0.001). (D) Tau protein and tubulin co-localization in mouse primary cortical neurons incubated with vehicle (DMSO) or the VASH specific inhibitor LV-80. (E) Automated quantification of tau immunofluorescent staining in tubulin-positive regions (n >150). P-values were calculated with the multiple t-test (^{∗∗∗}<0.001). (F) Quantification of tubulin in mouse primary cortical neurons incubated with vehicle (DMSO) or the VASH specific inhibitor LV-80. Automated quantification of immunofluorescent signal in positive regions (n >150). P-values were calculated with the multiple t-test (ns; not significant).

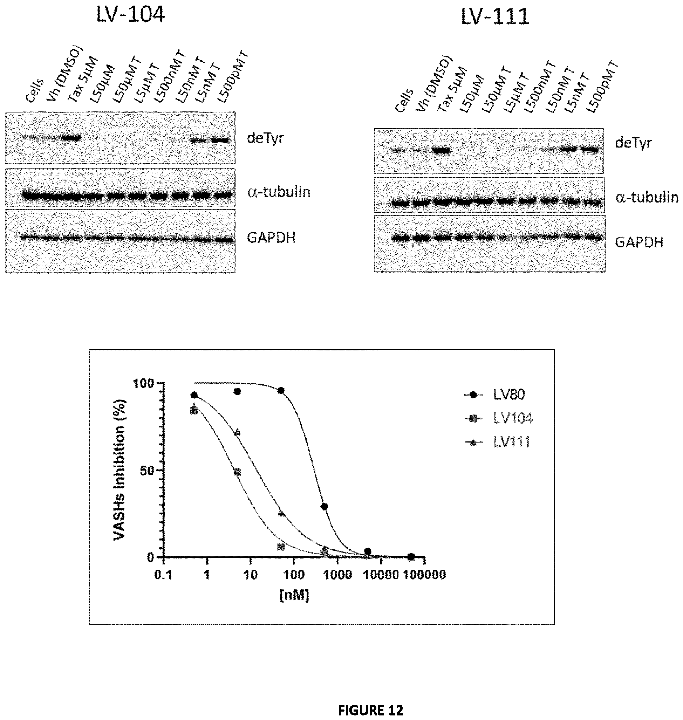

*Figure 12**:* Newly developed prodrug (LV-104 and LV-111) display very efficient inhibition of VASH-dependent detyrosination in human CHL-1 cells.

### Examples

### 1) Synthesis

### Abbreviations:

Aad : α-aminoadipic acid
All : Allyl group
BOP : benzotriazol-1-yloxytris(dimethylamino)phosphonium haxefluorophosphate
DCM : Dichloromethane
DIEA: N,N-Diisopropylethylamine
DMF : dimethylformamide
DMSO : dimethylsulfoxide
HATU : 1-[Bis(dimethylamino)methylene]-1H-1,2,3-triazolo[4,5-b]pyridinium 3-oxide hexafluorophosphate
NMP : N-methyl-2-pyrrolidone
PyAOP : 7-Azabenzotriazol-1-yloxy)tripyrrolidinophosphonium hexafluorophosphate
RM : reaction mixture
RT : room temperature (18°C-25°C)
SPPS : solid phase peptide synthesis
TES : Trans epoxy succinate
TFA : trifluoroacetic acid
THF : tetrahydrofuran
TiS : triisopropylsilane
Tyr : Tyrosine

### 1.1) Material

All of the Fluorenylmethyloxycarbonyl (Fmoc) protected amino acids and 1-[Bis(dimethylamino)methylene]-1H-1,2,3-triazolo[4,5-b]pyridinium3-oxid hexafluorophosphate (HATU) were provided by Iris Biotech GmbH. Piperidine, N,N-Diisopropylethylamine (DIEA), TFA, Triisopropylsilane (TIS), dichloromethane (DCM), 1,2-Dichloroethane (DCE), N,N-Dimethylformamide (DMF), N-methylpyrrolidinone (NMP) and ethyl ether (Et₂O) were provided by Sigma Aldrich. AmphiSpheres 40 RAM 0.38 mmol/g 75-150 µm resin was purchased from Agilent Technologies. Solvents used for HPLC and LC/MS were of HPLC grade.

### SPPS procedure

Peptide synthesis was performed using a standard SPPS protocol. Each synthesis was performed using Fmoc-Rink amide AmphiSpheres 40 resin (0.37 mmol/g). The Fmoc protected amino acids (4 eq), HATU (4 eq) and DIEA (6 eq) were added to the syringe reactor and the mixture was stirred for 1 hour at room temperature. After each coupling reaction, the peptideresin was submitted to two 5 min deprotection cycles with DMF/piperidine 80/20 v/v solution.

### Compounds purification

All crude compounds were purified by preparative HPLC (Waters 4000 apparatus) on a C18 reversed-phase column (C18 Deltapak column, 100 mm × 40 mm, 15 µm, 100 Å) at a flow rate of 50 mL/min of a H2O + 0.1% TFA and CH3CN + 0.1% TFA mixture in gradient mode with UV detection at 214 nm. Fractions containing the pure product were collected and lyophilized.

### LC/MS Analyses

Samples were prepared in an acetonitrile/water (50/50 v/v) mixture containing 0.1% TFA. The LC/MS system consisted of a Waters Alliance 2690 HPLC coupled to a Micromass (Manchester, UK) ZQ spectrometer (electrospray ionization mode, ESI+). All analyses were carried out using a C18 Chromolith Flash 25 × 4.6 mm column. A flow rate of 3 ml/min and a gradient of 0-100% Acetonitrile over 5 min of a H2O + 0.1% HCOOH and CH3CN + 0.1% HCOOH mixture in gradient mode with UV detection at 214 nm. Positive-ion electrospray mass spectra were acquired at a solvent flow rate of 100-200 µl/min. Nitrogen was used for both the nebulizing and drying gas. The data were obtained in a scan mode ranging from 200 to 1700 m/z in 0.1 s intervals; ten scans were summed up to obtain the final spectrum. Retention times are given in minutes. Solvents used for HPLC and LC/MS were of HPLC grade.

### 1.2) Synthesis and characterization

### 1.2.1) Synthesis of compounds of formula (I) according to the present invention

### Intermediate A synthesis

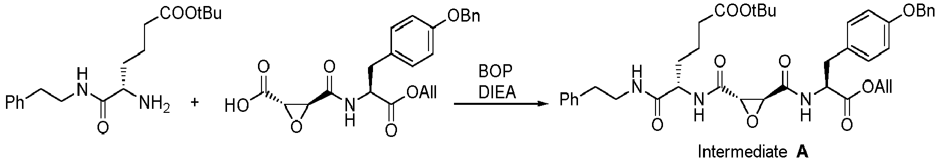

tert-butyl (S)-5-amino-6-oxo-6-(phenethylamino)hexanoate (131mg, 0.41mmol) and HO-TES-Tyr(OBn)-OAII (174mg, 0.41mmol) were solubilized in DMF followed by the addition of DIEA (139µl, 0.82mmol) and BOP (173mg, 0.41mmol). After 2 hours, the product was isolated by extraction. The obtained white powder was then dried over night at 0.2mbar to afford 260mg of intermediate A as white solid.
*Expected mass: 260mg* / *Obtained mass: 300mg* / *Crude Yield: 86%*
*t_{R} = 2.18, MS (ESI+): m*/*z= 728.4 [M+H]⁺*

### Synthesis of LV-80

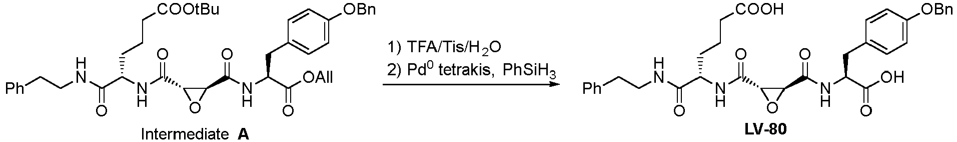

Intermediate A (100mg, 0.137mmol) was dissolved in 15ml of DCM, followed 15ml of TFA/TiS/H₂O. The RM was then stirred for 30min at RT. The RM was concentrated dry and the remaining oil was precipitated with cold EtOEt. The precipitate was filtered and dried over reduced pressure. Next, the precipitate was dissolved in 1ml of dry DMF and the allyl ester was removed by treatment with Pd⁰ Tetrakis (5.15mg, 0.0045mmol) and PhSiH₃ (37µl, 0.3mmol). After 20 min the RM was dissolved in 20ml EtOAc that was washed 1 time with 1M HCI solution, 1 time with brine and concentrated dry. The obtained precipitate was then purified by preparative HPLC to obtain 41mg of **LV-80** as white solid.
*Expected mass: 91mg* / *Obtained mass: 41mg* / *Crude Yield: 45%*
*t_{R} = 3.10, MS (ESI+): m*/*z=* 632.4 *[M+H]⁺*

### Synthesis of LV-86

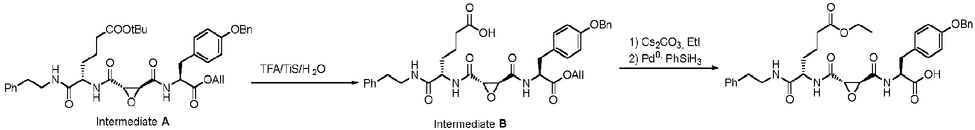

Intermediate **A** (100mg, 0.137mmol) was dissolved in 15ml of DCM, followed 15ml of TFA/TiS/H₂O. The RM was then stirred for 30min at RT. The RM was concentrated dry and the remaining oil was precipitated with cold Et₂O. The precipitate was filtered and dried over reduced pressure to obtain Intermediate **B**.

Intermediate **B** (15mg, 0.022 mmol) was dissolved in NMP, followed by the addition of cesium carbonate (7.15mg, 0.022 mmol) and iodoethane (2.34µl, 0.029 mmol). The RM was then stirred at RT for 1 hour. Next, the RM was poured in 30ml of water. The aqueous phase was extracted 3 times with EtOAc. The regrouped organic phases were washed 3 times with saturated NaHCO₃, 3 times with KHSO₄ 1M then washed with brine, dried over Na₂SO₄ and evaporated dry to afford 17mg white solid. Next, the white solid was dissolved in 1ml of dry DMF and the allyl ester was removed by treatment with Pd⁰ Tetrakis (1mg, 0.00087mmol), PhSiH₃ (3µl, 0.024mmol). After 20 min the RM was dissolved in 20ml EtOAc that was washed 1 time with 1M KHSO₄ solution, 1 time with brine and concentrated dry. The obtained precipitate was then purified by preparative HPLC to obtain 8,1mg of **LV-86** as white solid.
*Expected mass: 15,8mg* / *Obtained mass: 8,1mg*/*Crude Yield: 51%*
*t_{R} = 3.49, MS (ESI+): m*/*z= 660 [M+H]⁺*
¹H NMR (500 MHz, DMSO) δ 8.70 (s, 1H), 8.60 (s, 1H), 8.18 (t, *J* = 5.5, 1H), 7.44 (d, *J* = 7.5, 2H), 7.38 (t, *J* = 7.4, 2H), 7.32 (t, *J* = 7.3, 1H), 7.27 (t, *J* = 7.5, 2H), 7.21 - 7.16 (m, 2H), 7.14 (d, *J* = 8.3, 2H), 6.93 (d, *J* = 8.2, 2H), 5.05 (s, 2H), 4.38 (d, *J* = 4.6, 1H), 4.23 (dd, *J* = 13.6, 7.6, 1H), 4.04 (q, *J* = 7.1, 2H), 3.57 (d, *J* = 5.1, 2H), 3.22 (td, *J* = 13.0, 6.9, 2H), 3.02 (dd, *J* = 13.8, 4.4, 1H), 2.84 (dd, *J* = 13.8, 9.4, 1H), 2.69 (t, *J* = 7.2, 2H), 2.24 (t, *J* = 7.2, 2H), 1.57 (m, *J* = 6.7, 1H), 1.50 (m, 1H), 1.45 (m, *J* = 16.4, 8.0, 3H), 1.17 (t, *J* = 7.1, 3H)
¹³C NMR (126 MHz, DMSO) δ 172.63, 172.50, 170.67, 165.65, 165.49, 157.11, 139.33, 137.20, 130.23, 129.57, 128.73, 128.46, 128.32, 127.85, 127.79, 126.15, 114.52, 69.15, 59.80, 53.95, 52.58, 52.46, 52.35, 50.63, 35.77, 35.04, 33.09, 31.52, 20.80, 14.17.

### Synthesis of LV-87

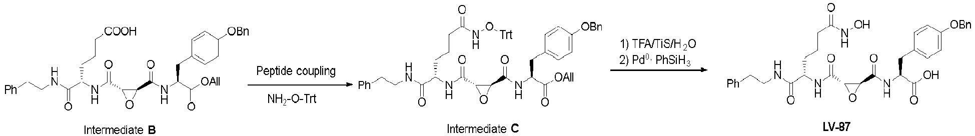

Intermediate **B** (10mg, 0.015 mmol) was dissolved in anhydrous THF. N-Methylmorpholine (3,82µL, 0.030 mmol) was added to the solution. The reaction mixture was then cooled down to -15 °C and stirred for 5 min. Isobutylchloroformate (1,94 piL, 0.015 mmol) was added, and the mixture was stirred for 10 min at -15 °C. O-Tritylhydroxylamine (8.25mg, 0.030 mmol) was added and the stirring continued for 15 min at -15 °C and 30 min at room temperature. Afterward, the mixture was poured into EtOAc (20 mL) washed 1M KSHO₄, saturated solution of NaHCO₃, dried over MgSO₄ and concentrated dry to afford 14mg of intermediate C as a white powder

### Expected mass: 14 mg / Obtained mass: 18mg / Crude Yield: quantitative

Intermediate C (13,92mg, 0.015mmol) was dissolved in 10ml of DCM, followed 10ml of TFA/TiS/H₂O. The RM was then stirred for 30min at RT. The RM was concentrated dry and the remaining oil was precipitated with cold EtOEt. The precipitate was filtered and dried over reduced pressure. Next, the precipitate was dissolved in 0,5ml of dry DMF and the allyl ester was removed by treatment with Pd⁰ Tetrakis (1mg, 0.00087mmol), PhSiH₃ (2,2µl, 0.0175mmol). After 20 min the RM was dissolved in 20ml EtOAc that was washed 1 time with 1M HCI solution, 1 time with brine and concentrated dry. The obtained precipitate was then purified by preparative HPLC to obtain 2 mg of **LV-87** as white solid.
*Expected mass: 11mg* / *Obtained mass: 2mg* / *Crude Yield: 17%*
*t_{R} = 2.93, MS (ESI+): m*/*z= 647.3 [M+H]⁺*
¹H NMR (500 MHz, DMSO) δ 8.80 (d, *J* = 8.0, 1H), 8.70 (s, 1H), 8.61 (d, *J* = 8.1, 1H), 8.17 (t, *J* = 5.6, 1H), 7.44 (d, *J* = 7.2, 2), 7.38 (d, *J* = 14.8, 2H), 7.32 (t, *J* = 7.3, 1H), 7.29 - 7.26 (m, 2H), 7.20 - 7.17 (m, 3H), 7.15 (d, *J* = 8.6, 2H), 6.94 (d, *J* = 8.7, 2H), 5.06 (s, 2H), 4.44 - 4.38 (m, 1H), 4.26 - 4.19 (m, 1H), 3.58 (s, 2H), 3.26 - 3.20 (m, 2H), 3.02 (dd, *J* = 13.8, 4.6, 1H), 2.84 (dd, *J* = 13.9, 9.4, 1H), 2.72 - 2.67 (m, 2H), 1.90 (dd, *J* = 13.0, 6.6, 2H), 1.60 - 1.53 (m, 1H), 1.49 - 1.43 (m, 2H), 1.42 - 1.37 (m, 1H).
¹³C NMR (500 MHz, DMSO) δ 172.50, 168.75, 165.75, 165.45, 157.14, 139.32, 137.18, 130.20, 128.71, 128.45, 128.35, 127.84, 127.78, 126.15, 114.43, 69.14, 53.82, 52.53, 52.37, 48.50, 35.07, 31.98, 31.79, 30.14, 21.63.

### Synthesis of LV-81

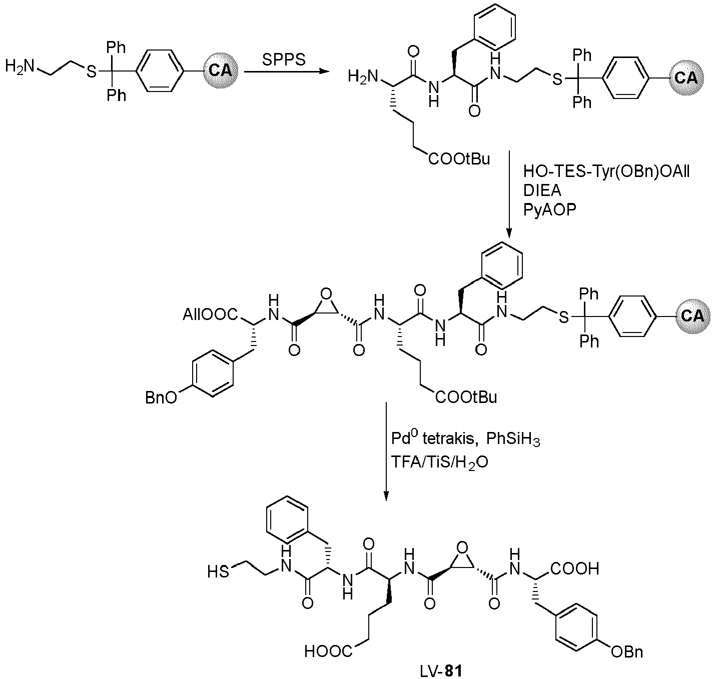

The H-Aad(tBu)Phe- sequence was synthetized via SPPS on cystamine-Trt resin (CA) charged 0.5 mmol/g. Next, the solid supported sequence was attached via an amide bond to compound HO-TES-Tyr(OBn)-OAII (95mg, 0.225mmol) via PyAOP (95mg, 0.225mmol) and DIEA (77µl, 0.450mmol) activation, overnight at RT. After 2 hours, Pd Tetrakis (8.66mg, 0.008mmol) and PhSiH₃ were added, leading to the deprotection of the OAII ester. The thiolated version of **LV-80** was cleaved from the resin by a 2 hours long treatment with TFA/DCM/H₂O/TiS 50/50/2.5/2.5. The filtrate was then concentrated dry to provide a brown oil that was precipitated in cold Et₂O to obtain a white solid that was isolated by filtration. The compound was then purified by preparative HPLC to obtain 32mg of **LV-81** as white solid.

### Expected mass: 55mg / Obtained mass: 32mg / Crude Yield: 58%

### Intermediate D synthesis

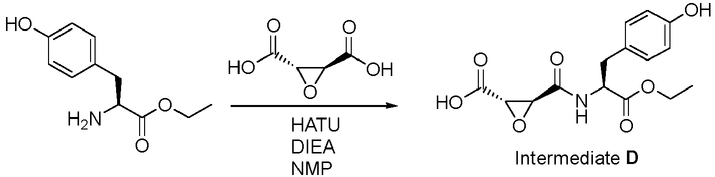

HCI.H-Tyr-OEt (100mg,0.407mmol) and (2S,3S)-trans-Oxirane-2,3-dicarboxylic acid (80.8mg, 0.611mmol) were dissolved in 1.5mL of NMP, followed by the addition of DIEA (1,628 mmol, 374µL). The mixture was stirred and HATU (309,5mg, 0,814mmol) was added. The mixture was stirred at room temperature for 20 min. The reaction mixture was poured in a Na₂CO₃ 1 M solution and extracted 3 times with 30 mL of diethyl ether. The aqueous phase was acidified with KHSO₄ 1M and the resulting solution was extracted 3 times with 30 mL of ethyl acetate. The organic layers were combined, washed with brine then dried over MgSO₄, filtered and concentrated under reduced pressure to afford intermediate D as a yellow oil. The product was used as it is without further purification.
Expected mass: 131mg / Obtained mass: 104mg / Yield: 79,3%
HPLC purity = 98%, tR =1.2min, MS (ESI+): m/z= 324,3 [M+H]+ , (expected m/z = 324.31)

### Synthesis of LV-111

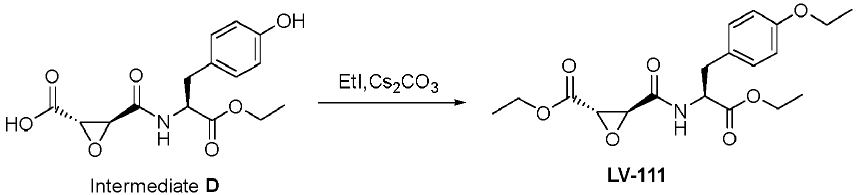

Cs₂CO₃ (314mg, 0.966mmol) was added to Intermediate D (104mg, 0.321mmol) dissolved in anhydrous DMF and the reaction was stirred for 15min. lodoethane (77.6µL, 0,966mmol) was added to the solution, and the reaction was stirred at room temperature overnight and monitored by HPLC. The solution was poured in 30mL of water and extracted 3 times with 30mL of ethyl acetate then washed 3 times with a solution of NaHCO₃ 1 M and 3 times with a solution of KHSO₄ 1 M. The organic layer was dried over MgSO₄, filtered, and concentrated under reduced pressure to afford the desired product as white crystals. The product was then purified by preparative chromatography to obtain 51.5mg of **LV-111** as white solid.
*Expected mass: 121.66mg* / *Obtained mass: 51.5mg* / *Yield: 42%*
HPLC purity = 98%, t_{R} =2.1min, MS (ESI+): m/z= 380.2 [M+H]⁺, (expected m/z = 380.16)
¹H NMR (500 MHz, DMSO) δ 8.78 (d, J = 7.9, 1H), 7.07 (d, J = 8.3, 2H), 6.78 (d, J = 8.2, 2H), 4.42 (dd, J = 14.2, 8.4, 1H), 4.17 - 3.89 (m, 6H), 3.61 (s, 1H), 3.40 (s, 1H), 2.93 (dd, J = 13.8, 5.7, 1H), 2.83 (dd, J = 13.7, 9.2, 1H), 1.29 - 1.05 (m, 9H).
¹³C NMR (126 MHz, DMSO) δ 171.40,167.43, 165.49, 157.81, 130.66, 128.90, 114.60, 63.32, 62.06, 61.24, 54.20, 53.06, 51.69, 36.17, 15.14, 14.34.
HRMS (micrOTOF-Q): calculated for C₁₉H₂₅NO₇ [M+H]+ : 380.1631, found: 380.1704.

### Synthesis of LV-101

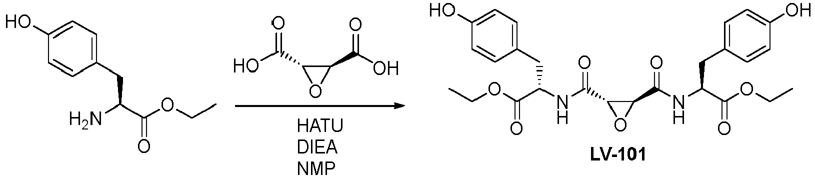

HCI.H-Tyr-OEt (100mg,0.407mmol) and (2S,3S)-trans-Oxirane-2,3-dicarboxylic acid (27mg, 0.204mmol) were dissolved in 1.5mL of NMP, followed by DIEA (1,628 mmol, 374µL). The mixture was stirred for 5 min and HATU (154,7mg, 0,407mmol) was added. The mixture was stirred at room temperature for 20 min. The solution was extracted 3 times with ethyl acetate. The organic layers were washed 3 times with KHSO₄ 1M and NaHCO₃ 1 M, then one time with brine and dried over MgSO₄, filtered and concentrated under reduced pressure to afford the desired product as a white powder.
HPLC purity = 98%, t_{R} =1.42min, MS (ESI+): m/z= 515.20 [M+H]+ , (expected m/z = 515.20)

### Synthesis of LV-104

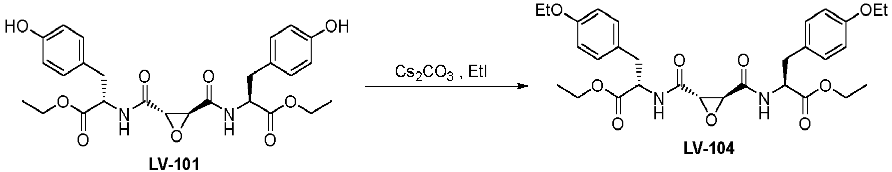

Cs₂CO₃ (126.8mg, 0.389mmol) was added to **LV-101** (100mg, 0.195mmol) dissolved in anhydrous DMF and the reaction was stirred for 15min. lodoethane (47.0µL, 0,585mmol) was added to the solution, and the reaction was stirred at room temperature overnight and monitored by HPLC. The solution was poured in 30mL of water and extracted 3 times with 30mL of ethyl acetate then washed 3 times with a solution of NaHCO₃ 1 M and 3 times with a solution of KHSO₄ 1 M. The organic layer was dried over MgSO₄, filtered, and concentrated under reduced pressure to afford the desired product as a white powder. The product was then purified by preparative chromatography to obtain 75.6mg of **LV-104** as white powder.
*Expected mass: 111.15mg* /*Obtained mass: 75.6mg* / *Yield: 68%*
HPLC purity = 99%, t_{R} =3.81min, MS (ESI+): m/z= 571.3 [M+H]⁺, (expected m/z = 571.26)
¹H NMR (500 MHz, DMSO) δ 8.87 (d, J = 7.7, 2H), 7.07 (d, J = 8.2, 4H), 6.78 (d, J = 8.1, 4H), 4.38 (dd, J = 14.2, 8.3, 2H), 4.02 (q, J = 7.1, 4H), 3.93 (q, J = 6.9, 4H), 3.45 (s, 2H), 2.92 (dd, J = 13.8, 5.7, 2H), 2.82 (dd, J = 13.8, 9.2, 2H), 1.25 (t, J = 6.9, 6H), 1.08 (t, J = 7.1, 6H). ¹³C NMR (126 MHz, DMSO) δ 170.97, 165.50, 157.29, 130.25, 128.36, 114.14, 62.70, 60.62, 53.79, 52.17, 35.51, 14.62, 13.91.
HRMS (micrOTOF-Q): calculated for C₃₀H₃₈N₂O₉ [M+H] + : 571.6390, found: 571.2650

### Synthesis of Intermediate E

Carbonyldiimidazole CDI (113.6mg ; 1.2eq) was dissolved in a minimum of DMF and the RM was placed at -10°C. Boc-NH-NH₂ (92.5mg ; 1.2eq) was also dissolved in a minimum of DMF and added to the RM which was then stirred during 30min at -10°C. After this, TFA.HTyr(OEt)OEt (203.6mg; 1eq) was also dissolved in a minimum of DMF and was added to the RM followed by Et₃N (168µL). The RM was then stirred at room temperature during 2h50. For the work up step, distilled water and AcOEt were added in the RM and placed in a separation funnel. The organic phase was then washed with HCl 1M (x2), saturated NaHCO₃ (x2) and brine (x2). The solution obtained was then dried with MgSO₄, filtered and concentrated to give an oil (154.3mg ; Yield : 56%). This obtained oil was solubilized in a TFA/TIS/H₂O (95/2.5/2.5) solution and stirred at room temperature during 30min and then concentrated dry to give Intermediate E as crude oil. The compound was used without any further purification.

### Synthesis of LV-119

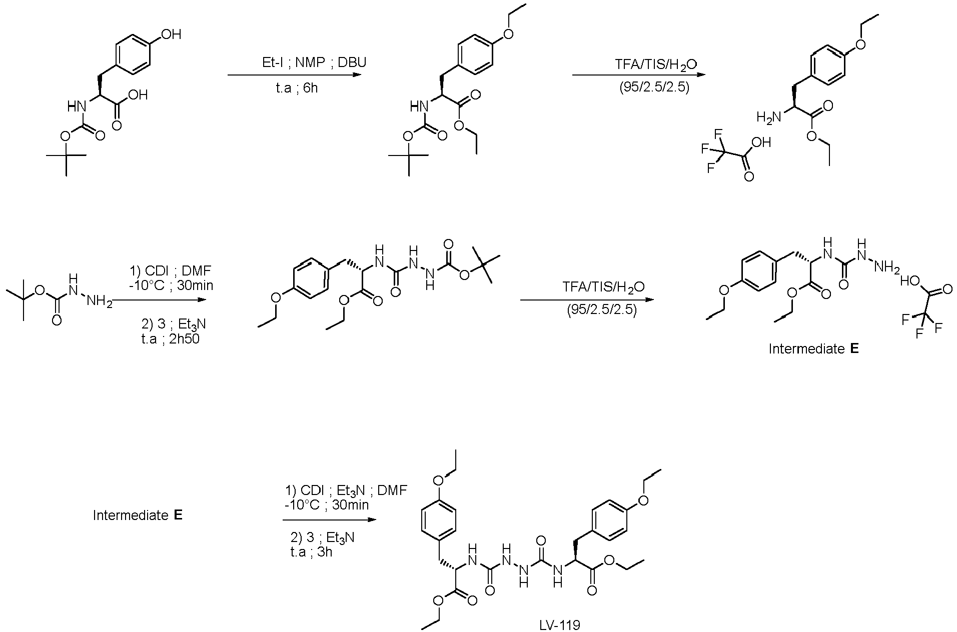

CDI (63.3mg ; 1.2eq) was dissolved in a minimum of DMF and the RM was placed at -10°C. Intermediate E (159.7mg ; 1.2eq) was also dissolved in a minimum of DMF and added to the RM which was then stirred during 30min at -10°C. The pH was adjusted to 8 with few drops of Et₃N. After this, TFA.HTyr(OEt)OEt (115.9mg ; 1eq) was also dissolved in a minimum of DMF and was added to the RM followed by Et₃N (102.2µL; 2.2eq). The RM was then stirred at room temperature during 3h. For the work up step, distilled water and AcOEt were added in the RM and placed in a separation funnel. The organic phase was then washed with HCl 1M (x2), saturated NaHCO₃ (x2) and brine (x2). The solution obtained was then dried with MgSO₄, filtered and concentrated to give an oil.

The raw product was then purified on HPLC and lyophilized to give very light and white crystals (43.9mg ; Yield : 24% ; Purity : >96%).
¹H NMR(500MHz, CDCl3) δ 6.99 (d,J = 8.5, 4H), 6.75 (d,J = 8.6, 4H), 6.50 (s, 2H), 6.05 (d,J = 8.1, 2H), 4.60 (dd,J = 14.3, 6.6, 2H), 4.16 - 4.04 (m, 4H), 3.92 (q,J = 7.0, 4H), 3.03 - 2.89 (m, 4H), 1.38 - 1.28 (m, 6H), 1.18 (t,J = 7.1, 6H).

### 1.2.2) Synthesis of conjugate of formula (I') according to the present invention

### Synthesis of LV-82

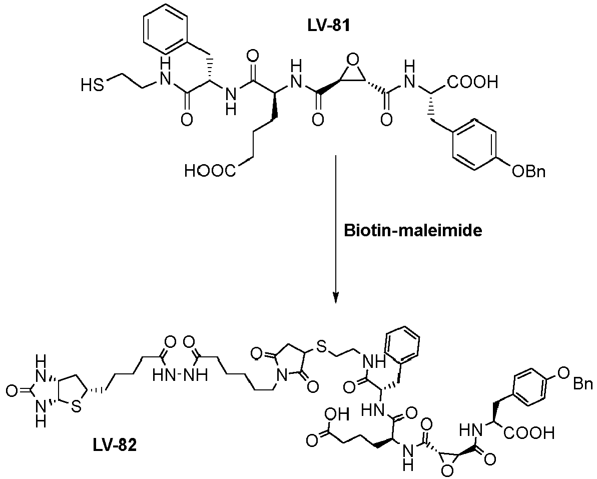

Compound **81** (7.35mg, 0.01mmol) was dissolved in 800µl of DMF followed by the addition of biotin-maleimide (4.5mg, 0.01mmol) and 400µl PBS solution. The mixture was lightly heated in order to solubilize both reagents and then stirred at RT for 1h. The RM was then directly purified by preparative HPLC to afford 7mg of **LV-82** as white solid.
*Expected mass: 11.85mg* / *Obtained mass: 7mg* / *Yield: 59%*
*t_{R} = 2.89, MS (ESI+): m*/*z= 1186.2 [M+H]⁺, 593.7 [M+2H]²⁺*

### 2) Biological results

### 2.1) Experimental Model

### Cell culture

Human CHL-1 cells (melanoma derived cell line, Nieuwenhuis J. et al. Science, 2017, 358(6369):1453-1456) were routinely cultured in DMEM containing 10% (v/v) fetal bovine serum (FBS), 50 U/ml penicillin and 50 µg/ml streptomycin (pen/strep) and incubated at 37°C. Prior testing, the CHL-1 cells were seeded in adequate plates (96,48,24,12 or 6 wells) at 30% confluency. Next day cells were treaed and collected for further analysis (immunoblotting, qPCR, immunofluorescence...).

Cortical cell cultures were seeded either on glass coverslips or plastic plates coated with 0.1 mg/ml poly-L-lysine (Sigma) at ^{~}10⁵ cells per cm² or 2×10⁴ cells per cm² for low-density cultures. Neurons were plated in DMEM containing 10% (v/v) fetal bovine serum (FBS), 50 U/ml penicillin and 50 µg/ml streptomycin (pen/strep), and 1-2 h later the medium was replaced with Neurobasal medium supplemented with 2% B27, pen/strep, 0.6% glucose and 1% Glutamax (all reagents from Life Technologies).

### Drosophila Melanogaster

Drosophila were raised at 25°Celsius. yw flies were used as control foryw; αTub84BΔ3 mutant. Total males were disrupted in laemli loading buffer, boiled, sonicated and loaded for immunoblotting.

### 2.2) Method Details

### Protein expression and purification

We used the methods as disclosed in the application WO2020/012002.

Human VASH1 (hVASH1) was cloned in the expression vector with a poly-histidine tag. Bacteria were transformed and induced with Isopropyl β-d-1-thiogalactopyranoside (IPTG) overnight or for 4 hours. Bacteria were collected and disrupted using a HTU-DIGI-F press (Heinemann). Recombinant proteins were purified using nickel-based affinity chromatography (IMAC) according to the manufacturer's protocol (GE Healthcare).

### In vitro detyrosination assays

*Spodoptera frugiperda* Sf9 cells were grown, lysed and used for tubulin purification by affinity chromatography. Microtubules were obtained with taxol and stored until use. *In vitro* analysis of detyrosination activities was performed using the recombinant hVASH1. Detyrosination assays were performed in the presence of 0.5 µM microtubules. Reactions were stopped by addition of the denaturing loading buffer followed by 5min incubation at 95°C, and samples were loaded on SDS PAGE for immunoblot analysis.

### Deqfutamyfation assays and tubulin purification

Deglutamylation assays have been performed as previously described (Rogowski et al., Cell, 2010, 143(4):564-78).

### Transfection of human cells

Plasmid transfections were performed and cells were collected 24 hours after transfection for immunoblotting and immunofluorescence analysis.

### Quantitative PCR

Total RNA was isolated with TRIzol (Invitrogen). Reverse transcription was carried out with random hexanucleotides. Quantitative PCR assays were performed using the Lightcycler SYBR Green Master mix on a Lightcycler apparatus (Roche). All used primers were intron-spanning. The relative amount of target cDNA was obtained by normalization by geometric averaging of multiple internal control genes.

### Immunofluorescence labeling

Methanol-fixed CHL-1 cells and mouse cortical neurons were analyzed using a Zeiss Axioimager Apotome microscope after standard immunofluorescence experiments. Briefly, samples were incubated with primary antibodies overnight, washed three times in PBS before 1h incubation with the following secondary antibodies: Alexa Fluor 488-conjugated goat antirat, goat anti-mouse and Alexa Fluor 555-conjugated goat anti-rabbit (Invitrogen). After incubation, samples were washed three times in PBS, stained with DAPI, and mounted.

### Microscopy

Microscopy image acquisition was performed at the Montpellier RIO Imaging facility and images were processed using OMERO.

### 2.3) Results

### 2.3.1 In cellulo VASH-mediated tubulin detyrosination assay

We used human CHL-1 cells in which tubulin detyrosination is catalyzed exclusively by VASHs. In these cells, detyrosination is predominantly VASH-dependent (Nieuwenhuis J. et al. Science, 2017, 358(6369):1453-1456). Cells were pre-treated with taxol before incubation with Epo-Y. Samples were immunoblotted using the indicated antibodies.

By using CHL-1 cells, we analyzed the use of Epo-Y to reduced taxol induced detyrosination. Cells were routinely cultured in a standard humidified tissue culture incubator at 37°C in presence of 5% CO2 and plated in a 6-wells culture dish. The cells were treated for 2 hours with Taxol in absence or presence of Epo-Y.

The cells were collected in a RIPA buffer (of 50 mM Tris HCI, 150 mM NaCl, 1.0% (v/v) NP-40, 0.5% (w/v) Sodium Deoxycholate, 1.0 mM EDTA, at a pH of 7.4), and quantitation of total protein performed using BCA kit (Thermo Fisher Scientific). A 20µg protein sample of a total cell extract was run on 10% SDS-PAGE, transferred to nitrocellulose, and probed with each antibody. Western blot analysis showed a striking decrease of taxol treated (2 hours) and consequent tubulin detyrosination in CHL-1 cells. As illustrated in **Figure 1**, incubation of CHL-1 cells with taxol led to a marked increase in tubulin detyrosination and acetylation levels, most likely due to MT stabilization. Moreover, the tubulin detyrosination activity was dosedependently inhibited by Epo-Y, a previously described VASH inhibitor.

So this *in cellulo* screening assay with CHL-1 cells and taxol is a relevant screening assay for identification of putative inhibitor of VASH-metiated tubulin detyrosination.

### 2.3.2 Putative inhibitor parthenolide does not inhibit VASH-mediated tubulin detyrosination

To study the inhibitory effects of parthenolide (PTL) on VASH-mediated tubulin detyrosination, we used the previously developed *in vitro* detyrosination assay disclosed in WO2020/012002.Reactions were incubated with inhibitor, stopped and analysed by immunoblotting with the indicated antibodies. As illustrated in **Figure 2****,** addition of 10 µM Epo-Y to the reaction mixture containing MTs resulted in complete inhibition of VASH1-mediated tubulin detyrosination **(****Figure 2B****).** Conversely, in the same assay, addition of much higher PTL concentrations (50 and 100 µM) did not result in any detectable inhibition of VASH1-dependent tubulin detyrosination **(****Figure 2B****).** To further extend our analysis, we used human CHL-1 cells in which tubulin detyrosination is catalysed exclusively by VASHs (Nieuwenhuis J. et al. Science, 2017, 358(6369):1453-1456). In these cells, detyrosination is predominantly VASH-dependent (Nieuwenhuis J. et al. Science, 2017, 358(6369):1453-1456). Cells were pre-treated with taxol before incubation with Epo-Y or PTL. Samples were immunoblotted using the indicated antibodies. Incubation of CHL-1 cells with taxol led to a marked increase in tubulin detyrosination and acetylation levels, most likely due to MT stabilization **(****Figure 2C****).** This allowed to directly compare Epo-Y and PTL inhibitory potency in cells incubated with taxol. As expected, Epo-Y reduced tubulin detyrosination in a dosedependent manner, without affecting tubulin acetylation. In agreement with the *in vitro* data **(****Figure 2B****),** tubulin detyrosination was not inhibited by PTL, even at the highest concentration used (Figure 2C). We concluded that PTL is not an inhibitor of VASH-mediated tubulin detyrosination.

### 2.3.3 Newly developed inhibitors target VASH1 and VASH2 and peptidase activity

**Figure 3** illustrates example of the inhibitory efficiency of various newly designed VASH inhibitors on both VASH1 and VASH2-mediated tubulin detyrosination at concentrations ranging from 0.25-05 µM.

### 2.3.4 Cell penetrant potent inhibitors of VASH peptidase activity

A small, highly reactive epoxide-based molecule such as Epo-Y might interact with free thiols present on proteins other than the VASHs or with nucleophilic lysine residues in the active sites of tyrosine kinases. Therefore, through iterative chemical variations and analysis with our standardized *in vitro* assay, we identified a compound, which we termed LV-43 **(****Figure 4A****).** Samples were immunoblotted using the indicated antibodies. As illustrated, LV-43 has more potent *in vitro* inhibition of VASH1 enzymatic activity than Epo-Y **(****Figure 4B****).** However, when tested in a cell-based assay involving taxol treated CHL-1 cells, LV-43 was much less efficient then Epo-Y **(****Figure 4C****).** The discrepancy between the *in vitro* and *in cellulo* data suggests reduced cell penetration of LV-43 compared with Epo-Y. Therefore, we generated a new series of compounds and added hydrophobic aromatic functional groups to improve cell permeability and enzyme affinity. In the *in vitro* assay, the indicated inhibitors were added to the reaction mixture, and then detyrosination was analysed by immunoblotting with the indicated antibodies. We found that LV-80 inhibited VASH1-mediated tubulin detyrosination more potently than LV-43 and Epo-Y, as indicated by their *in vitro* IC50 (28nM for LV80 *versus* 320nM for Epo-Y) **(****Figure 4D-E****).** Determination of the IC50 *in cellulo* using our taxol-induced detyrosination assay in CHL-1 cells confirmed the greater potency of LV-80 than Epo-Y also in cells (676nM for LV-80 versus 3µM for Epo-Y) **(****Figure 4F****).** Finally, using the available structural data of VASH1, we performed molecular docking to visualize the most likely binding mode of the LV-80 inhibitor, taking into consideration the formation of hydrogen bonds **(****Figure 4H****).** In summary, we designed and generated a specific cell-penetrant VASH inhibitor with an *in vitro* IC50 in the low nanomolar range, suitable for functional analysis of tubulin detyrosination.

### 2.3.5 Complete inhibition of VASH-mediated tubulin detyrosination in cells

To test the LV-80 potency on the endogenous detyrosination activity, we compared wild type CHL-1 cells incubated with LV-80 to double knockout CHL-1 cells (2KOs) that lack both VASH1 and VASH2. Cells were collected and analysed by immunoblotting with the indicated antibodies. After 24-hour incubation with LV-80, the level of detyrosination was comparable in treated CHL-1 and CHL-1 2KOs cells **(****Figure 5****),** as indicated by western blot analysis. This demonstrated the efficacy of the newly designed compound to completely block VASH-mediated detyrosination *in cellulo.* We also analysed tubulin detyrosination by immunofluorescence in cells incubated with vehicle LV-80. We confirmed the absence of signal for detyrosinated tubulin in cells treated with the inhibitor (data not shown).

### 2.3.6 Advantageous safety profile of VASH enzyme inhibitors

We next choose a widely used MTT-based colorimetric assay to assess the potential metabolic toxicity and cell viability alterations in the presence of LV-80. The MTT-based assay (3-[4,5-dimethylthiazol-2-yl]-2,5 diphenyl tetrazolium bromide) was performed 48 hours after incubation with the indicated compounds to monitor cell viability.

Incubation for 24 hours with 0.1µM taxol and 10µM PTL resulted in a significant reduction of cell viability. Conversely, incubation with a much higher concentration of LV-80 (100µM) had no significant effect on cell viability, which was similar to that of cells incubated with vehicle **(****Figure 6A****).** Analysis of mitochondrial function with a widely recognized and well-accepted assay did not show any significant change in oxygen consumption rate (OCR) and extracellular acidification rate (ECAR) in cells incubated with LV-80, underscoring the excellent safety profile of this compound **(****Figure 6B****).**

### 2.3.7 Newly developed VASH inhibitors are specific to VASH enzymatic activity

Next, we tested LV-80 inhibitory activity towards cytosolic carboxypeptidases (CCPs), another family of proteases that target tubulin C-terminal tails and act as tubulin deglutamylases. The inhibitor did not affect CCP1 and CCP5 activities. Samples were immunoblotted using the indicated antibodies. When polyglutamylated brain tubulin was incubated in the presence of protein lysates from HEK293 cells transfected with GFP-CCP1, we observed that CCP1-dependent deglutamylation activity towards long glutamate chains was not affected by addition of LV-80 **(****Figure 7A****).** Similar outcome was observed when assessing CCP5-mediated deglutamylation of branching point glutamate residues using this assay **(****Figure 7B****).** Overall, these findings demonstrated LV-80 specificity for detyrosinating VASH activity. Then, we analysed LV-80 specificity by testing its effect on the activity of carboxypeptidase A (CPA), an enzyme widely used for *in vitro* detyrosination of MTs and tubulin. The VASH-specific inhibitor did not affect carboxypeptidase A (CPA)-dependent tubulin detyrosination. On the other hand, the specific CPA inhibitor did not affect VASH activity. Indeed, addition of 10µM LV-80 resulted in complete loss of both VASH1- and VASH2-mediated detyrosination *in vitro,* but had no detectable effect on CPA activity **(****Figure 7C****).** Conversely, the peptidomimetic benzylsuccinic acid (BzISA) completely abolished CPA-dependent detyrosination, but not VASH1- or VASH2-mediated detyrosination.

### 2.3.8 Biotinylated version of newly developed inhibitor as research tool

Since detyrosination levels are particularly high in brain, we tested whether LV-80 fused to biotin could be used to pull down VASH1 from human brain extracts. Cells were pre-treated with taxol before incubation with biotinylated LV-80. Samples were immunoblotted using the indicated antibodies First, we confirmed that biotinylated LV-80 retained its inhibitory properties towards VASH1- and VASH2-mediated detyrosination *in vitro* **(****Figure 8A****),** and validated the pull-down methodology using wild type and 2KOs CHL-1 as controls *(**Figure **8B****).* Pull-down experiments were performed with protein lysates obtained from wild type and double VASH knockout cells. HEK293 cell protein lysate was loaded as control. Samples were immunoblotted using the indicated antibodies. Next, using human brain protein extracts, we efficiently pulled down VASH1 (***Figure 8C***), demonstrating the target engagement in a relevant human tissue. Input, flow through (FT) and pull down (Pd) fractions were immunoblotted using antibodies against the indicated proteins.

### 2.3.9 Strong reduction of tubulin detyrosination in primary cortical neurons treated with VASH inhibitor

We decided to assess the functional role of tubulin detyrosination in differentiating primary cortical neurons purified from cerebral cortical tissue of mouse embryos. After dissociation and filtration, we differentiated cells in the presence or absence of LV-80 for 7 days. Cells were collected and analysed by immunoblotting with the indicated antibodies. Samples were analysed by immunoblotting and the relative optical density measured in three independent assays (n = 3 and error bars represent SEM). P-values were calculated with the multiple t-test (n = 3, ^{∗}< 0.05, ^{∗∗}<0.01, ^{∗∗∗}<0.001, ^{∗∗∗∗}<0.0001). Analysis of the level of tubulin posttranslational modifications using specific antibodies **(****Figure 9A-B****)** showed that incubation of primary neurons with LV-80 resulted in a marked decrease of tubulin detyrosination levels, with a concomitant increase in tyrosination **(****Figure 9B****).** In the presence of LV-80, tubulin detyrosination was reduced by 70% and tyrosinated tubulin increased by 50% compared to control (DMSO), a slight difference reflecting the individual affinity of the antibodies. Decreased level of tubulin detyrosination is not due to an alteration of VASH1/2 or SVBP expression as assessed by qRT-PCR **(****Figure 9C****)** but results of direct inhibition of VASH enzymatic activity. Then, we analysed the differentiation status of neurons incubated with LV-80 or vehicle by qPCR quantification of a panel of neuronal differentiation and glial markers. The mRNA levels of Dcx and Neurod1, markers of early neuronal differentiation, and of Syp, Map2 and Dlg4, markers of later differentiation stages, were comparable between conditions **(****Figure 9D****).** Similarly, Sox2 and Gfap expression levels (glial markers) were comparable in both conditions **(****Figure 9E****).**

### 2.3.10 Detyrosination renders tubulin permissivie to glutomylation

Samples were analysed by immunoblotting and the relative optical density measured in three independent assays (n = 3 and error bars represent SEM). P-values were calculated with the multiple t-test (n = 3, ^{∗}< 0.05, ^{∗∗}<0.01, ^{∗∗∗}<0.001, ^{∗∗∗∗}<0.0001).

Strikingly, while acetylation levels remained unaffected in treated neurons **(****Figure 10B****),** polyglutamylation was strongly reduced **(****Figure 10A****),** as demonstrated with two different polyglutamylation-specific antibodies: PolyE, which recognizes long glutamate chains, and GT335, which is specific for branching point glutamate residues. This observation suggested the existence of a crosstalk between tubulin detyrosination and polyglutamylation. To test this hypothesis, we co-overexpressed wild type or enzymatically inactive VASH2 (VASH2D) with the TTLL4 or TTLL6 glutamylase in HEK293 cells. HEK293 cells were co-transfected with wild type VASH2 or a dead variant (VASH2D) and the TTLL4 or TTLL6 glutamylase. Cells were collected and analysed by immunoblotting with antibodies against the indicated modifications. Increased tubulin detyrosination levels stimulated TTLL6 but not TTLL4 polyglutamylation activity **(****Figure 10C****).** This demonstrated that TTLL6-activity is sensitive to the presence of the C-terminal tyrosine residue on a-tubulin. This result showed that detyrosination renders tubulin permissive to some polyglutamylases and identified a crosstalk between these pathways. Next, we tested whether in Drosophila such crosstalk between tubulin detyrosination and polyglutamylation is also present. Both modifications are particularly prominent in males where they accumulate on sperm axonemes during spermatogenesis. Drosophila lack VASH homolog and detyrosination is catalysed by an unknown enzyme. To mimic detyrosination, we used flies engineered to express a truncated form of the major alpha-tubulin isotype aTub84B (aTub84BΔ3 mutant13) lacking the three last amino acid residues, including tyrosine. Samples were collected and analysed by immunoblotting with the indicated antibodies. aTub84BΔ3 males display a marked increase in polyglutamylation levels compared to wild type males **(****Figure 10D****).** Thus, C-terminal amino acid residues influence polyglutamylation level *in vivo.* These results are in agreement with the existence of a conserved crosstalk between detyrosination and polyglutamylation.

### 2.3.11 Tubulin detyrosination impacts tau protein binding in primary cortical neurons

To study the role of tubulin detyrosination in regulating the interaction of MTs with MT associated proteins (MAPs), we first completed differentiation of the cortical neurons for 5 days in absence of VASH inhibitor and followed by a treatment of 3 days. This approach allows for monitoring the cell distribution of tau protein independently of cell differentiation. Cells were collected and analysed by immunoblotting with the indicated antibodies. Incubation of cortical neurons with LV-80 for 3 days efficiently reduced tubulin detyrosination level and significantly increased tyrosinated tubulin level **(****Figure 11A-B****).** Samples were analysed by immunoblotting and the relative optical density measured in three independent assays (n = 3 and error bars represent SEM). P-values were calculated with the multiple t-test (n = 3, ^{∗}< 0.05, ^{∗∗}<0.01, ^{∗∗∗}<0.001). We confirmed the detyrosination-polyglutamylation crosstalk, as indicated by the significant reduction of the PolyE and GT355 signals in cells incubated with inhibitor **(****Figure 11C****).** Overall, tau levels did not significantly differ between conditions as confirmed by immunoblot **(****Figure 11A****)** and qRT-PCR quantification (not shown) of tau mRNAs (encoded by the Mapt gene). Next, we stained for tau and tubulin in vehicle-and LV80-treated neurons to perform a quantitative analysis. We selected areas with specific tubulin staining and measured the immunofluorescent signal of tau protein exactly in the same regions in vehicle- and inhibitor-treated primary cortical neurons. While tubulin staining and quantification was not significantly different between conditions, tau cellular distribution was significantly altered in projections **(****Figure 11D-F****)** and specifically reduced in cells incubated with our VASH inhibitor (data not shown). This suggests a role for tubulin detyrosination in regulating the interaction between MTs and tau protein. Altogether, we showed that inhibition of tubulin detyrosination in primary cortical neurons directly affects the localization of tau.

### 2.3.12. Newly designed low nM in cellulo VASH inhibitors

We designed a set of highly effective prodrugs efficient at low nM IC50 *in cellulo* (including examples as LV-104 and LV-111) and are cell penetrant, as illustrated in **Figure 12****.**

The compounds were added prior Taxol-treatment and abolished VASH-mediated detyrosination in low nM ranges in CHL-1 cells. Two examples of prodrug compounds (LV-104 and LV-111) and a direct VASH inhibitor. Both prodrugs did not have any effect on VASH1 and VASH2 in the context of *in vitro* assays.

Considering all these results, we propose that targeting VASH peptidase activity is a safe and well tolerated mechanism for treating neurodegenerative diseases like Alzheimer's disease but also cancer, muscular dystrophy and ciliopathies. Interestingly these compounds have strong effect on tubulin glutamylation and may also be used for reducing TTLL-dependent modifications of tubulin in e.g infantile onset neurodegeneration or glaucoma.

We showed that parthenolide, a molecule extensively used as an inhibitor of detyrosination, does not inhibit VASH activity neither *in vitro* nor *in cellulo.* This is in agreement with a recently published study, which demonstrated that the effect of parthenolide on detyrosination is indirect and most likely results from changes in microtubule dynamics caused by its covalent binding to tubulin (Hotta et al. Curr Biol. 2021, Volume 31, Issue 4, Pages 900-907). More importantly, we described medicinal chemistry-based optimization of the Epo-Y compound, which led to the development of a new highly specific and cell-penetrant VASH inhibitor. We showed that our newly developed compounds (active ingredients) have the ability to completely inhibit VASH activity and, in contrast to parthenolide, shows no detectable toxicity. Furthermore, by applying the VASH inhibitor to primary neuronal cultures, we have discovered previously unknown crosstalk between tubulin detyrosination and another important tubulin modification called polyglutamylation. The newly identified link between these two modifications has far-reaching consequences for the establishment of neuronal polarity and the localization of the main neuronal microtubule associated protein called tau. Overall these compounds hold strong potential for the use in pharmaceutical development and for research purposes.

## Claims

1. A compound of formula (I) : 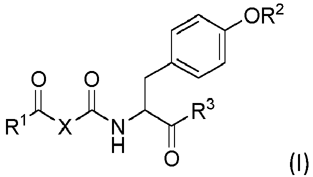 or a pharmaceutically acceptable salt and/or solvate thereof, in which
X is 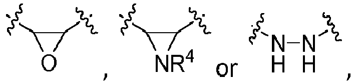
R¹ is O-C₁-C₆ alkyl, O-C₂-C₆ alkenyl, NH-OH or 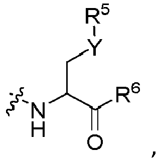
R² is H, a C₁-C₆ aliphatic chain, aryl, heteroaryl or C₁-C₆ alkyl-aryl, wherein up to 4 methylene units of said aliphatic chain are optionally replaced by O, C(O), NH or N-C₁-C₆alkyl, said aliphatic chain, aryl, heteroaryl or alkyl-aryl being optionally substituted,
R³ is OH, O-C₁-C₆ aliphatic chain, O-aryl, O-C₁-C₆ alkyl-aryl, O-heteroaryl, O-C₁-C₆ alkyl-heteroaryl or NHOH, wherein up to 4 methylene units of said aliphatic chain are optionally replaced by O, C(O), NH or, N-C₁-C₆alkyl, said aliphatic chain, aryl, heteroaryl, alkyl-heteroaryl or alkyl-aryl being optionally substituted,
R⁴ is H or a C₁-C₁₂ aliphatic chain wherein up to 4 methylene units are optionally replaced by O, C(O), NH or N-C₁-C₆alkyl, said C₁-C₁₂ aliphatic chain being optionally substituted,
Y is -(CH₂)ₘ- or 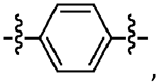
R⁵ is OH, O-C₁-C₆ alkyl, C₂-C₆ alkenyl, C₂-C₆ alkynyl, aryl, heteroaryl, O-C₁-C₆ alkyl-aryl, O-C₁-C₆ alkyl-heteroaryl, C(O)OH, C(O)O-C₁-C₆ alkyl or C(O)NHOH, said alkyl, alkenyl, alkynyl, aryl, heteroaryl, alkyl-aryl or alkyl-heteroaryl being optionally substituted,
R⁶ is OH, O-C₁-C₆ alkyl, NH-OH or NH-CH(R⁷)-(CH₂)ₙ-R⁸, said alkyl being optionally substituted, R⁷ is H, C₁-C₆ alkyl, C₂-C₆ alkenyl, C₂-C₆ alkynyl, aryl, heteroaryl, C₁-C₆ alkyl-aryl or C₁-C₆ alkyl-heteroaryl,
R⁸ is C(O)NH₂, C(O)NH-C₁-C₆ alkyl, aryl, heteroaryl, SH, NH₂ or S-C₁-C₆alkyl, and
m and n are each independently an integer ranging from 0 to 6, provided that when X is 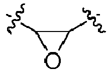 and R³ is OH, R¹ is not O-C₁-C₆ alkyl.

2. The compound according to claim 1, wherein R² is H, an optionally substituted C₁-C₆ alkyl or an optionally substituted C₁-C₆ alkyl-aryl.

3. The compound according to claim 1 or 2, wherein R³ is OH, O-C₁-C₆ aliphatic chain or NHOH, said aliphatic chain being optionally substituted.

4. The compound according to any one of claims 1 to 3, wherein R¹ is O-C₁-C₆ alkyl, O-C₂-C₆ alkenyl or NH-OH.

5. The compound according to any one of claims 1 to 3, wherein R¹ is 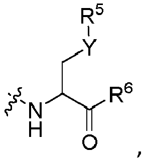 preferably 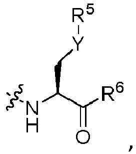 with Y, R⁵ and R⁶ being as defined in claim 1.

6. The compound according to claim 5, wherein Y is 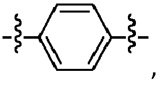 R⁵ is OH, O-C₁-C₆ alkyl, such as O-methyl or O-ethyl, O-C₁-C₆ alkyl-aryl, such as O-benzyl, and R⁶ is OH or O-C₁-C₆ alkyl, such as O-methyl or O-ethyl.

7. The compound according to claim 5, wherein Y is -(CH₂)ₘ- , preferably -(CH₂)₂-, R⁵ is C(O)OH, C(O)OEt or C(O)NHOH, R⁶ is NH-CH(R⁷)-(CH₂)ₙ-R⁸, R⁷ is H, C₁-C₆ alkyl, C₁-C₆ alkyl-aryl, such as benzyl or C₁-C₆ alkyl-heteroaryl, such as CH₂-indole group, R⁸ is C(O)NH₂, aryl, such as phenyl, heteroaryl, such as indole, SH, or S-C₁-C₆alkyl, such as S-C(CH₃)₃ and n is 0, 1, 2 or 3.

8. The compound according to any one of claims 1 to 7, wherein X is 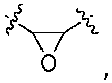 preferably

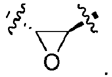

9. The compound according to any one of claims 1 to 8, responding to the following formula (I-A') :

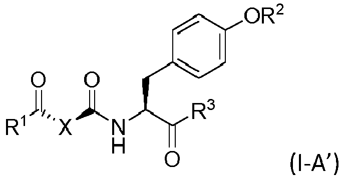

10. The compound according to claim 1, being selected from the following compounds : 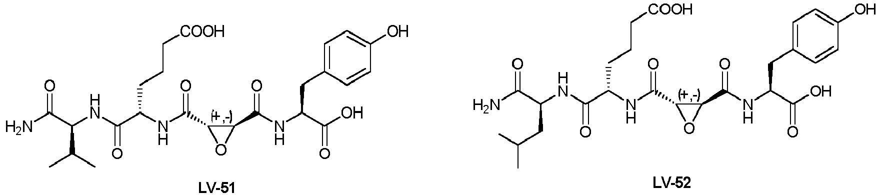 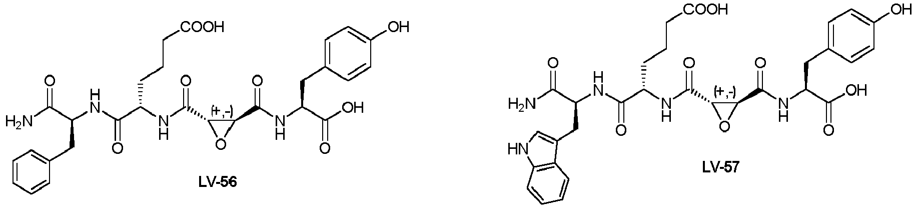 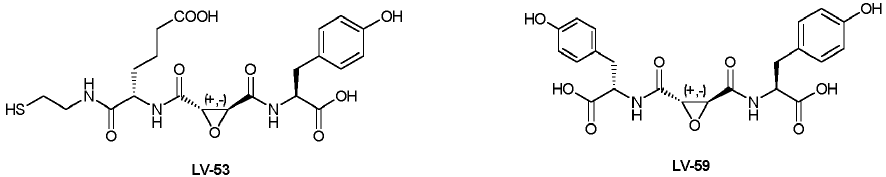 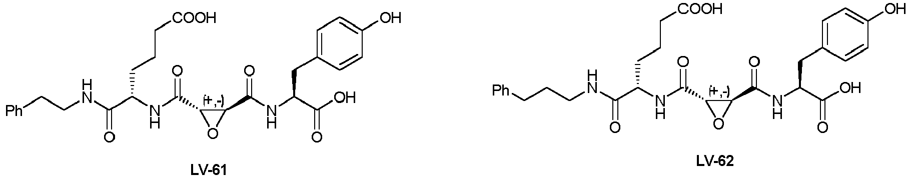 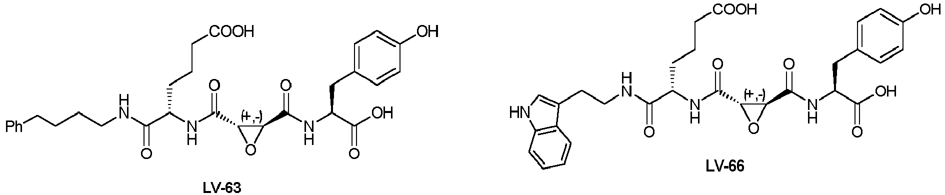 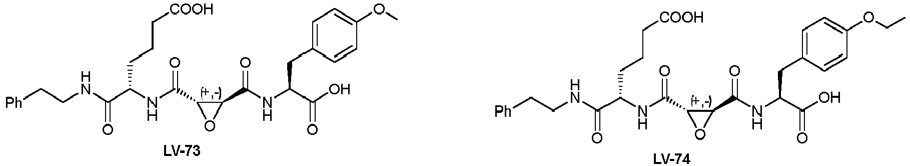 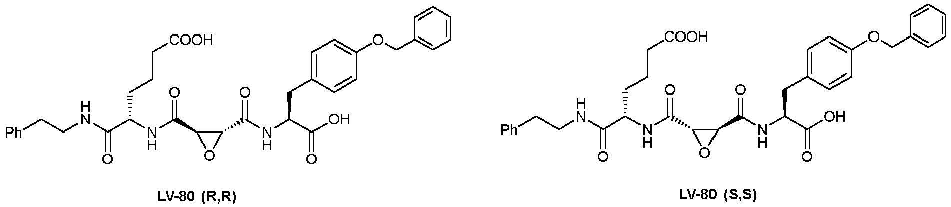 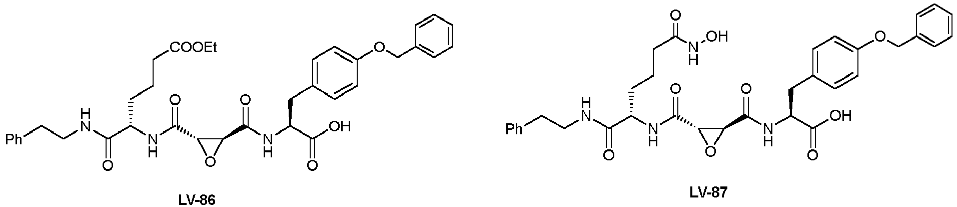 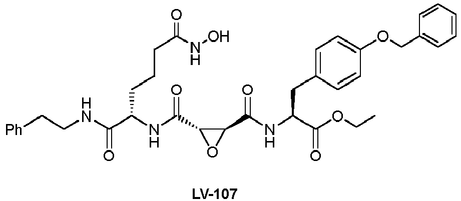 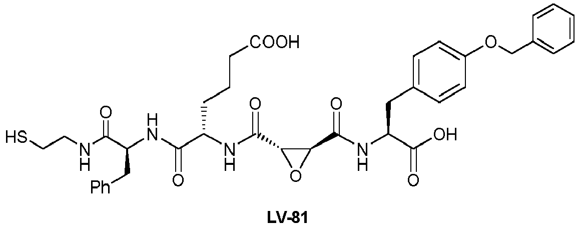 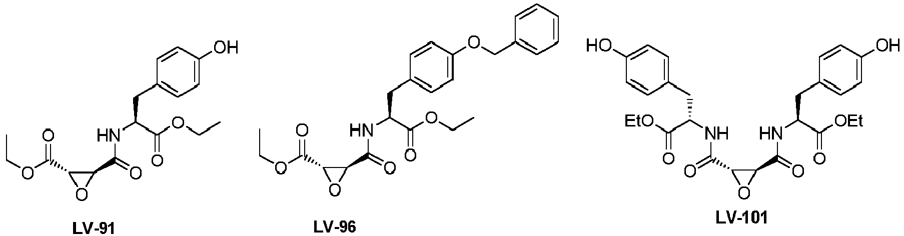 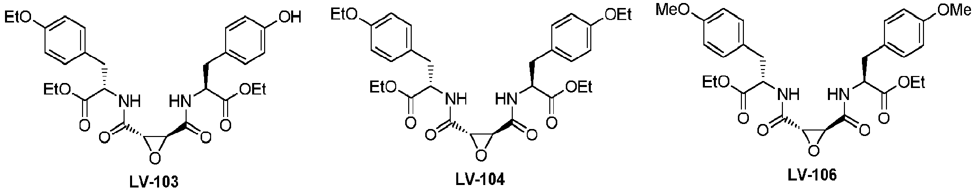 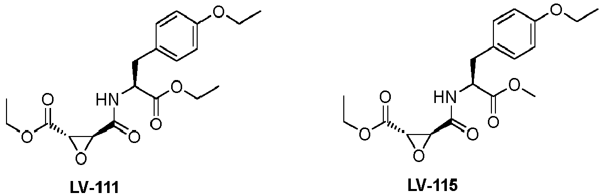 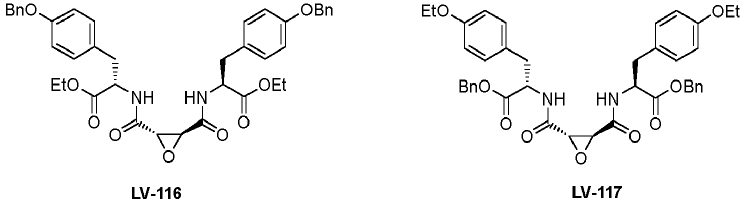 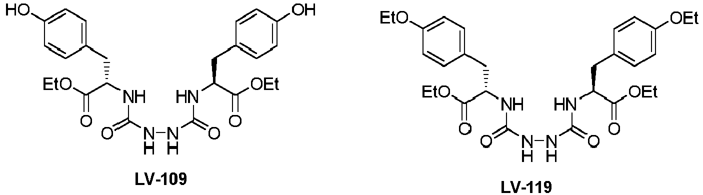 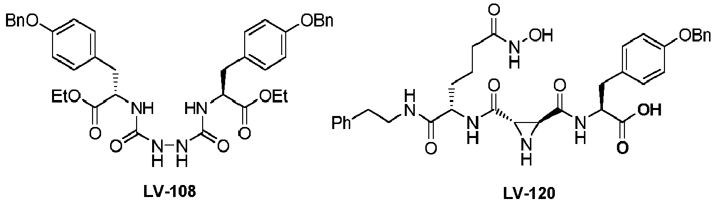 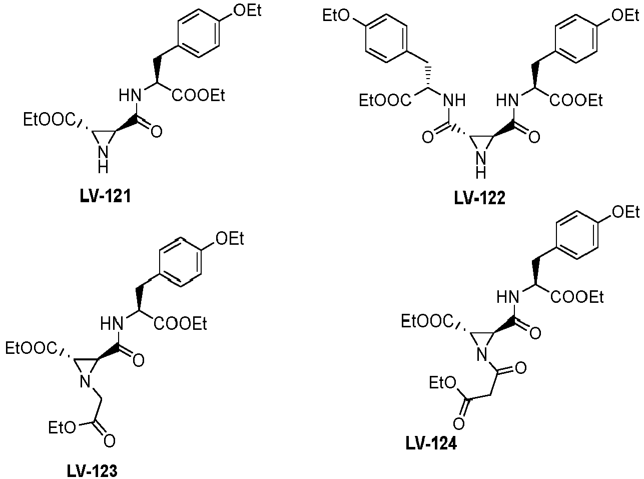 and the pharmaceutically acceptable salts and/or solvates thereof.

11. A pharmaceutical composition comprising at least one compound of formula (I) as defined in any one of claims 1 to 10 and at least one pharmaceutically acceptable excipient.

12. A compound according to any one of claims 1 to 10 or a pharmaceutical composition according to claim 11 for use as a drug.

13. A compound according to any one of claims 1 to 10 or a pharmaceutical composition according to claim 11 for use in the treatment of a VASH peptidase activity associated disorder.

14. A compound or a composition for use according to claim 13, wherein the VASH peptidase activity associated disorder is a disorder involving altered microtubule detyrosination and/or polyglutamylation, notably selected from neurodegenerative diseases, such as Alzheimer disease or Parkinson disease, glaucoma, psychiatric disorders, neuronal disorders, cancers, such as colon cancer and neuroblastoma, muscular dystrophies, infertility, retinal degeneration, Purkinje cell sicknesses, infantile onset degeneration, male infertilities and ciliopathies, in particular neurodegenerative diseases, cancers and muscular dystrophies.

15. A conjugate comprising a fragment of a compound of formula (I) as defined in any one of claims 1 to 10 linked to a biomolecule such as a peptide, a protein, a biomarker, as for example a photolabelling agent, such as Rhodamine, Coumarin Derivatives, cyanines derivatives or fluoresceine, an affinity probe such as biotin, or a E3 ubiquitin ligase recruiter such as Thalidomide, VH032, VH101, dBET1, dFKBP12, QCA570, PROTAC6, ZNL-02-096 or d9A-2.

16. A conjugate according to claim 15 for use as a drug.

17. A compound according to anyone of claims 1 to 10 or a conjugate according to claim 15 for use as research tool for research and development activities, in particular selected in the group consisting of:
- *in vitro* and/or *in cellulo* screening assays for identifying new VASH inhibitors and/or quantifying their inhibitor efficiency,
- *in vitro* method for studying the role of tubulin detyrosination, in particular in VASH peptidase activity associated disorder, such as its occurrence, aggressiveness and progression,
- *in vitro* diagnostic methods for identifying or monitoring a VASH peptidase activity associated disorder involving altered microtubule detyrosination and/or polyglutamylation,
- and related kits for performing said screening assays and methods.
